# EUROPÄISCHE PATENTSCHRIFT

(11) **EP 0 885 204 B1**
(45) Veröffentlichungstag und Bekanntmachung des Hinweises auf die Patenterteilung: **12.06.2002**
(21) Anmeldenummer: 97906150.4
(22) Anmeldetag: 03.03.1997
(51) Int. Cl.: C07D 295/14, C07D 213/74, C07D 317/60

(54) **NEUE ARYLGLYCINAMIDDERIVATE, VERFAHREN ZU IHRER HERSTELLUNG UND DIESE VERBINDUNGEN ENTHALTENDE PHARMAZEUTISCHE ZUSAMMENSETZUNGEN**
NOVEL ARYL GLYCINAMIDE DERIVATIVES, METHOD OF PRODUCING SAID DERIVATIVES AND PHARMACEUTICAL COMPOSITIONS CONTAINING THESE COMPOUNDS
NOUVEAUX DERIVES D'ARYLGLYCINAMIDE, LEUR PROCEDE DE FABRICATION ET COMPOSITIONS PHARMACEUTIQUES CONTENANT CES COMPOSES

(30) Priorität: 06.03.1996 DE 19608665
(43) Veröffentlichungstag der Anmeldung: 23.12.1998
(73) Patentinhaber: Boehringer Ingelheim Pharma KG, 55218 Ingelheim am Rhein (DE)
(72) Erfinder: ESSER, Franz, D-55218 Ingelheim am Rhein (DE); SCHNORRENBERG, Gerd, D-88400 Biberach (DE); SCHROMM, Kurt, D-55218 Ingelheim am Rhein (DE); DOLLINGER, Horst, D-55218 Ingelheim am Rhein (DE); JUNG, Birgit, D-55270 Schwabenheim (DE); SPECK, Georg, D-55218 Ingelheim am Rhein (DE)
(86) Internationale Anmeldenummer: EP9701038
(87) Internationale Veröffentlichungsnummer: WO9732865

(56) Entgegenhaltungen:
- DE-A- 19 519 245
- US-A- 3 033 869

## Beschreibung

US 3033869A offenbart α piperidino-α-phenyl-N-cyclohexylacetamide mit spasmolytischen Wirkung.

Die Erfindung betrifft neue Arylglycinamidderivate der allgemeinen Formel I 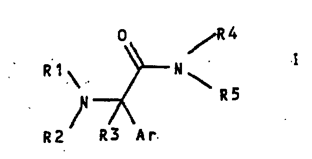 und deren pharmazeutisch annehmbare Salze,
Verfahren zu ihrer Herstellung und diese Verbindungen
enthaltende pharmazeutische Zusammensetzungen. Die
Verbindungen sind wertvolle Neurokinin
(Tachykinin)-Antagonisten.

Die in dieser Beschreibung und den Ansprüchen verwendeten Abkürzungen werden nachfolgend erklärt:
- CDI =: Carbonyldiimidazol
- DCCI =: Dicyclohexylcarbodiimid
- HOBt =: 1-Hydroxybenztriazol
- THF =: Tetrahydrofuran
- DMF =: Dimethylformamid
- RT =: Raumtemperatur
- DMAP =: 4-Dimethylaminopyridin
- TBTU =: O-Benzotriazolyl-tetramethyluronium-tetrafluoroborat

Für die Darstellung der Formeln wird eine vereinfachte Darstellung verwendet. Dabei werden in der Darstellung von Verbindungen z.B. alle CH₃-Substituenten jeweils durch einen Bindungsstrich dargestellt und CH durch ≡, so steht zum Beispiel 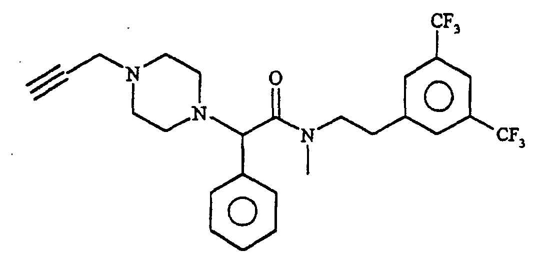 für

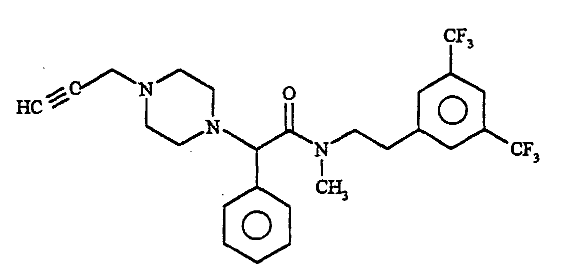

Die Erfindung betrifft neue Arylglycinamidderivate der allgemeinen Formel I 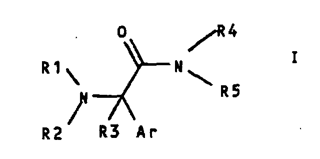 oder deren pharmazeutisch annehmbare Salze,
worin
- Ar: unsubstituiertes oder 1- bis 5-fach substituiertes Phenyl, oder unsubstituiertes oder 1- oder 2-fach substituiertes Naphthyl ist, [wobei die Substituenten des Phenyl und Naphthyl unabhängig voneinander Halogen (F, Cl, Br, J), (C₁-C₄)Alkyl, O-(C₁-C₄)Alkyl, CF₃, OCF₃ oder NR¹²R¹³ (worin R¹² und R¹³ unabhängig voneinander H, Methyl oder Acetyl sind) sind] oder Ar durch -O-CH₂-O- oder -O-(CH₂)₂-O- substituiertes Phenyl ist;
R¹ und R² zusammen mit dem N, an den sie gebunden sind, einen Ring der Formel 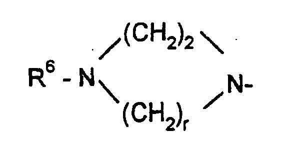 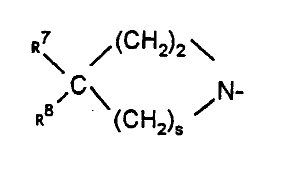 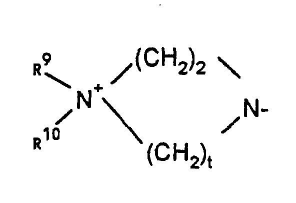 oder 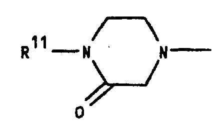 bilden, worin
r, s und t 2 oder 3 sind;
R⁶
H,
(C₁-C₅)Alkyl,
(C₃-C₅)Alkenyl,
Propinyl,
Hydroxy(C₂-C₄)alkyl,
Methoxy(C₂-C₄)alkyl,
Di(C₁-C₃)alkylamino(C₂-C₄)alkyl,
Amino(C₂-C₄)alkyl,
Amino,
Di(C₁-C₃)alkylamino,
Monofluor- bis Perfluor(C₁-C₂)alkyl,
N-Methylpiperidinyl,
Pyridyl,
Pyrimidinyl,
Pyrazinyl,
Pyridazinyl
oder die Gruppe -CH₂-C(O)NR¹⁴R¹⁵ bedeutet,
worin
R¹⁴ H oder (C₁-C₄)Alkyl ist und
R¹⁵ H, (C₁-C₄)Alkyl, (C₃-C₆)Cycloalkyl, Hydroxy(C₂-C₄)alkyl, Alkoxy(C₂-C₃)alkyl, Phenyl(C₁-C₄)alkyl, oder R¹⁴ und R¹⁵ zusammen mit dem N an den sie gebunden sind einen Ring (1-Pyrrolidinyl, Piperidino, Morpholino oder 1-Methylpiperazin-4-yl) bilden;
R⁷ eine der Bedeutungen (a) bis (d) hat,
(a) Hydroxy
(b) 4-Piperidinopiperidyl,
(c) 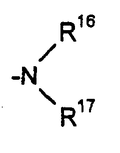 worin R¹⁶ und R¹⁷ unabhängig voneinander
   H,
   (C₁-C₄)Alkyl,
   (C₃-C₆)Cycloalkyl,
   Hydroxy(C₂-C₄)alkyl,
   (C₁-C₃)Alkoxy(C₂-C₄)alkyl,
   Phenyl(C₁-C₄)alkyl oder
   Di(C₁-C₃)alkylamino(C₂-C₄)alkyl sind,
   oder wenn R¹⁶ H oder (C₁-C₄)Alkyl ist,
   R¹⁷ auch -CH₂C(O)NR¹⁸R¹⁹ sein kann, worin R¹⁸ und R¹⁹
   wie obige R¹⁴ und R¹⁵ definiert sind;
(d) 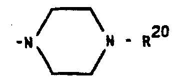 worin R²⁰
   H
   (C₁-C₄)Alkyl,
   (C₄-C₆)Cycloalkyl oder
   -CH₂C(O)NR²¹R²² ist,
   worin R²¹ und R²² wie obige R¹⁴ und R¹⁵ definiert sind;
R⁸ H ist
R⁹ und R¹⁰ unabhängig voneinander
(C₁-C₄)Alkyl sind;
R¹¹
H,
(C₁-C₅)Alkyl,
(C₃-C₅)Alkenyl,
Propinyl,
Hydroxy(C₂-C₄)alkyl,
Methoxy(C₂-C₃)alkyl,
Di(C₁-C₃)alkylamino(C₂-C₃)alkyl,
Amino(C₂-C₃)alkyl,
Amino,
Di(C₁-C₃)alkylamino,
Monofluor- bis Perfluor(C₁-C₂)alkyl,
N-Methylpiperidinyl,
Pyridyl,
Pyrimidinyl,
Pyrazinyl,
Pyridazinyl
oder die Gruppe -CH₂-C(O)NR²³R²⁴ bedeutet,
worin R²³ und R²⁴ wie obige R¹⁴ und R¹⁵ definiert sind;
- R3: H, (C₁-C₄)Alkyl, unsubstituiertes oder 1-3fach substituiertes Phenyl ist, worin die Substituenten unabhängig voneinander Halogen (F, Cl, Br, J), (C₁-C₄)Alkyl, O-(C₁-C₄)Alkyl, CF₃, OCF₃ oder NR²⁵R²⁶ (worin R²⁵ und R²⁶ unabhängig voneinander H, Methyl oder Acetyl sind) sind;
- R⁴: Phenyl(C₁-C₄)alkyl oder Naphthyl(C₁-C₄)alkyl bedeutet, worin Phenyl durch 1 bis 3 Substituenten substituiert sein kann, worin die Substituenten unabhängig voneinander Halogen (F, Cl, Br, J), (C₁-C₄)Alkyl, O-(C₁-C₄)Alkyl, CF₃, OCF₃ oder NR²⁷R²⁸ (worin R²⁷ und R²⁸ unabhängig voneinander H, Methyl oder Acetyl sind) sind;
und
- R⁵: H, (C₁-C₄)Alkyl, (C₃-C₆)Cycloalkyl, CH₂COOH, CH₂C(O)NH₂, OH oder Phenyl(C₁-C₄)alkyl bedeutet.

Bevorzugt sind Verbindungen der allgemeinen Formel 1,
worin
- Ar: unsubstituiertes oder 1- oder 2-fach substituiertes Phenyl, oder unsubstituiertes Naphthyl ist, oder Ar durch -O-CH₂-O- oder -O-(CH₂)₂-O- substituiertes Phenyl ist;

R¹ und R² zusammen mit dem N, an den sie gebunden sind, einen Ring der Formel 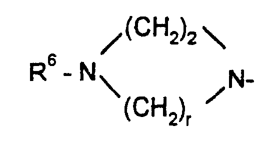 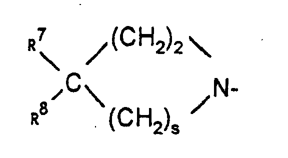 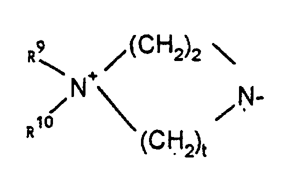 oder 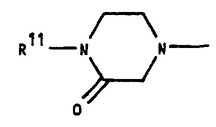 bilden, worin
r 2 oder 3 ist und
s und t 2 sind;
R⁶, R⁷, R⁸, R⁹, R¹⁰ und R¹¹ wie oben definiert sind;
- R³: H oder (C₁-C₄)Alkyl ist,
- R⁴: Phenyl(C₁-C₄)alkyl oder Naphthyl(C₁-C₄)alkyl bedeutet, worin Phenyl durch 1 oder 2 Substituenten substituiert sein kann, worin die Substituenten unabhängig voneinander Halogen (F, Cl, Br, J), (C₁-C₄)Alkyl, O-(C₁-C₄)Alkyl, CF₃ oder OCF₃ sind;
und
- R⁵: H, (C₁-C₄)Alkyl, (C₃-C₆)Cydoalkyl, OH oder (C₁-C₄)Alkylphenyl bedeutet.

Hervorzuheben sind Verbindungen der Formel I,
worin
- Ar: unsubstituiertes oder 1- oder 2-fach substituiertes Phenyl, oder unsubstituiertes Naphthyl ist, [wobei die Substituenten des Phenyl unabhängig voneinander Halogen (F, Cl, Br, J), Methyl, Methoxy, CF₃ oder OCF₃ sind] oder Ar durch -O-CH₂-O- oder -O-(CH₂)₂-O- substituiertes Phenyl ist;
insbesondere solche, worin Ar Phenyl, Naphthyl, in Position 3 und/oder 4 durch Methoxy oder Halogen substituiertes Phenyl ist oder Phenyl ist, dessen Positionen 2 und 3, oder 3 und 4 durch -O-CH₂-O- verbunden sind, vorzugsweise solche Verbindungen,
worin Ar Phenyl,
in Positionen 3 und 4 durch Methoxy substituiertes Phenyl ist
oder Phenyl ist, dessen Positionen 3 und 4 oder 2 und 3 durch
-O-CH₂-O- verbunden sind.

Von den oben definierten Verbindungen sind solche hervorzuheben,
worin in dem Ring 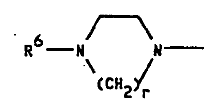 r 2 oder 3 ist, und
R⁶
H,
(C₁-C₅)Alkyl,
(C₃-C₅)Alkenyl,
Propinyl,
Hydroxy(C₂-C₄)alkyl,
Methoxy(C₂-C₄)alkyl,
Di(C₁-C₃)alkylamino(C₂-C₄)alkyl,
Amino(C₂-C₄)alkyl,
Amino,
Di(C₁-C₃)alkylamino,
Monofluor- bis Perfluor(C₁-C₂)alkyl,
N-Methylpiperidinyl,
Pyridyl,
Pyrimidinyl,
oder 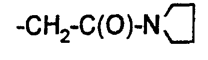 ist;
insbesondere solche, worin
r 3 ist und R⁶ Methyl;
und solche, worin
r 2 ist und
R⁶
H,
(C₁-C₄)Alkyl,
Propenyl,
Propinyl,
Hydroxy(C₂-C₃)alkyl,
Methoxyethyl,
Di(C₁-C₂)alkylamino(C₂-C₃)alkyl,
Aminoethyl,
Amino,
Dimethylamino,
CH₂ CF₃,
N-Methylpiperidinyl,
Pyridyl,
Pyrimidinyl,
oder 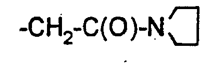 ist,
vorzugsweise solche, worin
r 2 ist und
R⁶ H, (C₁-C₃)Alkyl, Allyl, 2-Propinyl, -CH₂CH₂OCH₃,
-CH₂CH₂N(CH₃)₂, N-Methylpiperidinyl, 2-Pyrimidinyl
oder 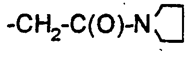 ist, insbesondere solche
worin
r 2 ist und
R⁶ H, CH₃, C₃H₇, CH(CH₃)₂,
CH₂CH₂OH, CH₂CH₂OCH₃ oder CH₂CH₂N(CH₃)₂.

Von den oben definierten Verbindungen sind ferner solche hervorzuheben, worin
R¹ und R² zusammen mit dem N, an den sie gebunden sind, den Ring 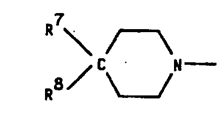 bilden,
worin R⁸ H ist und
R⁷
OH 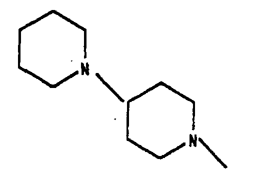 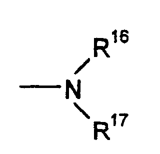 worin R¹⁶ und R¹⁷ unabhängig voneinander sind:
H
(C₁-C₃)Alkyl 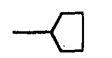 (CH₂)ₙ OH worin n 2, 3 oder 4 ist
(CH₂)₂ OCH₃
-(CH₂)ₙPh worin n 2 oder 4 ist
(CH₂)₂ N(CH₃)₂ 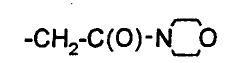 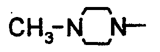 oder 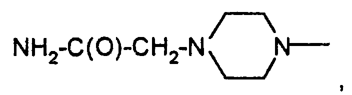 insbesondere solche, worin
R¹⁶ und R¹⁷ beide CH₃ oder C₂H₅ sind oder
R¹⁶ H oder CH₃ ist und R¹⁷
(C₁-C₃)Alkyl 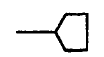 (CH₂)₂OH
(CH₂)₄OH oder 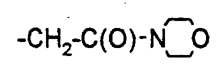 R⁷ 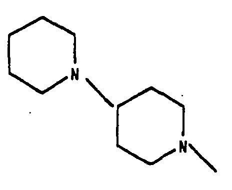 N(CH₃)₂ 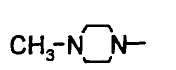 oder 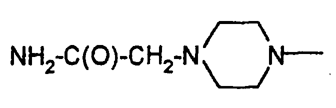 ist,
insbesondere solche,
worin
R¹ und R² zusammen mit dem N, an den sie gebunden sind, den Ring 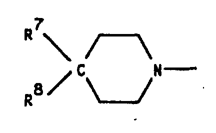 bilden,
worin
(a) R⁸ H ist und
   R⁷ 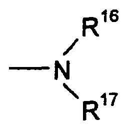 worin
   R¹⁶ und R¹⁷ beide CH₃, C₂H₅ oder
   CH₂CH₂OH sind oder
   R¹⁶ H oder CH₃ ist und R¹⁷
   (C₁-C₃)Alkyl 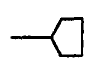 (CH₂)₂OH oder
   (CH₂)₄OH
   oder
(b) R⁸ H ist und
   R⁷

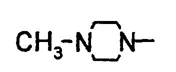

Von den oben definierten Verbindungen sind ferner solche hervorzuheben, worin
R¹ und R² zusammen mit dem N, an den sie gebunden sind,
den Ring 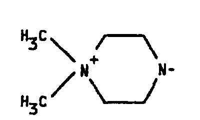 bilden.

Von den oben definierten Verbindungen sind ferner solche hervorzuheben, worin
R¹ und R² zusammen mit dem N, an den sie gebunden sind,
den Ring 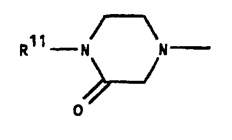 bilden, worin R¹¹ H oder (C₁-C₃)Alkyl ist, insbesondere solche
worin
R¹¹ -CH(CH₃)₂ ist.

Von den oben definierten Verbindungen sind solche von besonderem Interesse, worin R³ H ist;
und/oder
- R⁴: Phenyl(C₁-C₄)alkyl bedeutet, worin Phenyl durch 1 oder 2 Substituenten substituiert sein kann, worin die Substituenten unabhängig voneinander Halogen (F, Cl, Br, J), (C₁-C₄)Alkyl, O-(C₁-C₄)Alkyl, CF₃ oder OCF₃ sind;
und/oder
- R⁵: H, (C₁-C₄)Alkyl, (C₃-C₆)Cycloalkyl,
-OH oder Phenyl(C₁-C₄)alkyl bedeutet,
insbesondere solche, worin
R⁴ Phenyl(C₂-C₄)alkyl bedeutet, worin die Substituenten in den Positionen 3 und/oder 5 des Phenylringes sind und/oder
R⁵ H, Methyl, OH oder Phenethyl ist,
vorzugsweise solche, worin
R⁴ 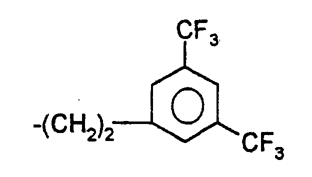 ist und R⁵ Methyl ist.

Verbindungen der allgemeinen Formel l können Säuregruppen besitzen , hauptsächlich Carboxylgruppen, und/oder basische Gruppen wie z.B. Aminofunktionen. Verbindungen der allgemeinen Formel l können deshalb entweder als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Die erfindungsgemäßen Verbindungen können als Racemate vorliegen, sie können aber auch als reine Enantiomere, d.h. in (R)- oder (S)-Form gewonnen werden.

Die oben verwendete Bezeichnung Naphthyl umfaßt sowohl 1-Naphthyl als auch 2 Naphthyl.

### Untersuchungsergebnisse für erfindungsgemäße Verbindungen:

Die Rezeptoraffinität zum NK₁-Rezeptor (Substanz P-Rezeptor) wird an humanen Lymphoblastoma-Zellen (IM-9) mit klonierten NK₁-Rezeptoren bestimmt, wobei die Verdrängung von ¹²⁵J-markierter Substanz P gemessen wird. Die so erhaltenen Kᵢ-Werte zeigen die Wirksamkeit der Verbindungen:

| | Ki [nM] |
|---|---|
| Beispiel 1 | 1,2 |
| Beispiel 2 | 1,0 |
| Beispiel 3 | 19 |
| Beispiel 4 | 1,4 |
| Beispiel 5 | 1,5 |
| Beispiel 8 | 1,8 |
| Beispiel 9 | 2,5 |
| Beispiel 11 | 3,8 |
| Beispiel 12 | 5,0 |
| Beispiel 13 | 2,4 |
| Beispiel 15 | 0,98 |
| Beispiel 16 | 0,90 |
| Beispiel 17 | 7,75 |
| Beispiel 18 | 0,96 |
| Beispiel 19 | 1,17 |
| Beispiel 20 | 2,0 |
| Beispiel 22 | 2,2 |
| Beispiel 23 | 2,5 |
| Beispiel 24 | 2,2 |
| Beispiel 25 | 6,0 |
| Beispiel 26 | 1,6 |
| Beispiel 28 | 1,3 |
| Beispiel 30 | 1,8 |
| Beispiel 32 | 1,3 |
| Beispiel 33 | 7,4 |
| Beispiel 34 | 2,9 |
| Beispiel 47 | 1,7 |
| Beispiel 55 | 1,25 |
| Beispiel 63 | 1,4 |
| Beispiel 64 | 1,1 |
| Beispiel 65 | 5,7 |
| Beispiel 73 | 2,0 |
| Beispiel 74 | 1,5 |
| Beispiel 75 | 0,44 |
| Beispiel 76 | 2,0 |

Die erfindungsgemäßen Verbindungen sind wertvolle Neurokinin (Tachykinin)-Antagonisten, die sowohl Substanz P-Antagonismus, als auch Neurokinin A- bzw. Neurokinin-B-antagonistische Eigenschaften besitzen. Sie sind nützlich zur Behandlung von und zur Vorbeugung gegenüber Neurokinin-vermittelten Krankheiten:
Behandlung oder Vorbeugung von entzündlichen und allergischen Erkrankungen der Atemwege, wie Asthma, chronische Bronchitis, hyperreagible Atemwege, Emphysem, Rhinitis, Husten,
der Augen, wie Konjunktivitis und Iritis,
der Haut, wie Dermatitis bei Kontaktekzem, Urtikaria, Psoriasis, Sonnenbrand, Insektenstiche, Juckreiz, sensible oder überempfindliche Haut,
des Magen-Darm-Traktes, wie Magen- und Duodenalgeschwüre, Colitis Ulcerosa, Morbus Crohn, Colon irritabile, M. Hirschsprung,
der Gelenke, wie rheumatoide Arthritis, reaktive
   Arthritis und Reiter-Syndrom;
zur Behandlung von Erkrankungen des Zentralnervensystems,
   wie Demenz, M. Alzheimer, Schizophrenie, Psychosen, Depression, Kopfschmerzen (z.B. Migräne oder Spannungskopfschmerzen), Epilepsie; M. Parkinson, Schlaganfall, Behandlung von Herpes zoster sowie postherpetischer Schmerzen, von Tumoren, Kollagenosen, einer Dysfunktion
   der ableitenden Hamwege, von Hämorrhoiden, von Übelkeit und Erbrechen, ausgelöst z.B. durch Bestrahlung oder Zytostatikatherapie oder Bewegung und Schmerzzuständen aller Art.

Die Erfindung betrifft daher auch die Verwendung der erfindungsgemäßen Verbindungen als Heilmittel und pharmazeutische Zubereitungen, die diese Verbindungen enthalten. Bevorzugt ist die Anwendung am Menschen. Die Applikation der erfindungsgemäßen Verbindungen kann intravenös, subcutan, intramuskulär, intraperitoneal, intranasal, inhalativ, transdermal, gewünschtenfalls durch lontophorese oder literaturbekannte Enhancer gefördert, und oral erfolgen.

Zur parenteralen Applikation werden die Verbindungen der Formel I oder deren physiologisch veträglichen Salze, eventuell mit den dafür üblichen Substanzen wie Lösungsvermittler, Emulgatoren oder weitere Hilfsstoffe in Lösung, Suspension oder Emulsion gebracht. Als Lösungsmittel kommen z.B. in Frage: Wasser, physiologische Kochsalzlösungen oder Alkohole, z.B. Ethanol, Propandiol oder Glycerin, Zuckerlösungen wie Glucose- oder Mannit-Lösungen oder auch eine Mischung aus verschiedenen Lösungsmitteln.

Außerdem können die Verbindungen durch Implantate, z.B. aus Polylactid, Polyglycolid oder Polyhydroxybuttersäure bzw. intranasale Zubereitungen appliziert werden.

Die orale Wirksamkeit von Verbindungen der allgemeinen Formel I kann durch folgenden Standardtest gezeigt werden:

Hemmung von durch NK₁ herbeigeführte Blutdrucksenkung in anästhesierten Meerschweinchen.

Meerschweinchen (300-500 Gramm) wurden mit Pentobarbital (50 mg/kg i.p.) anästhesiert, intubiert und mechanisch beatmet. Die Beatmung erfolgte mit jeweils 10 ml/kg Luft und einer Frequenz von 60 Atemzügen pro Minute. Die Halsschlagader wurde mit einer Kanüle versehen und der arterielle Blutdruck wurde verzeichnet. Ein Polyethylenschlauch wurde für intravenöse Zufuhr von Substanzen in die Halsvene eingeführt.

Eine zeitlich vorübergehende Blutdrucksenkung wurde in Intervallen von 10 Minuten herbeigeführt, indem intravenös der NK₁-Agonist
[βAla⁴, Sar⁹, Met(O₂)¹¹] SP(4-11) 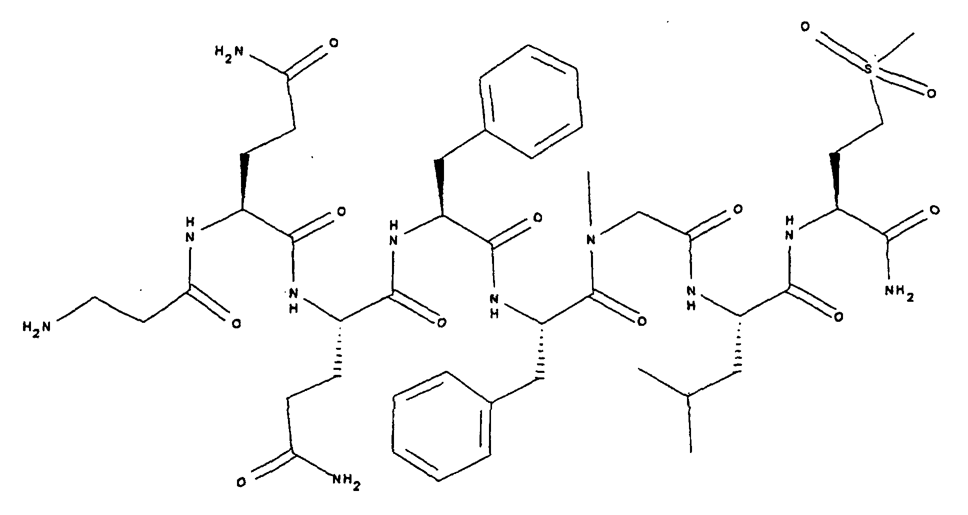 in einer Dosis von 0,2 µmol/kg verabreicht wurde. Nach Feststellung des so erzeugten Blutdruckes wurde die Testverbindung in den Zwölffingerdarm eingeführt und wieder wurde der NK₁-Agonist alle 10 Minuten injiziert.

Die Resultate wurden ausgedrückt in %-Hemmung der durch den genannten NK₁-Agonisten verursachten Blutdrucksenkung.

Die Verbindung des Beispiels 1 hemmte bei einer Dosis von 1 mg/kg (verabreicht in den Zwölffingerdarm) die durch den NK₁-Agonisten verursachte Blutdrucksenkung um 80%.

Die erfindungsgemäßen Verbindungen können nach allgemein bekannten Methoden hergestellt werden.

Die Herstellung der Verbindungen kann auf verschiedene Weise erfolgen.
Die beiden gebräuchlichsten Verfahren sind im folgenden Schema dargestellt:

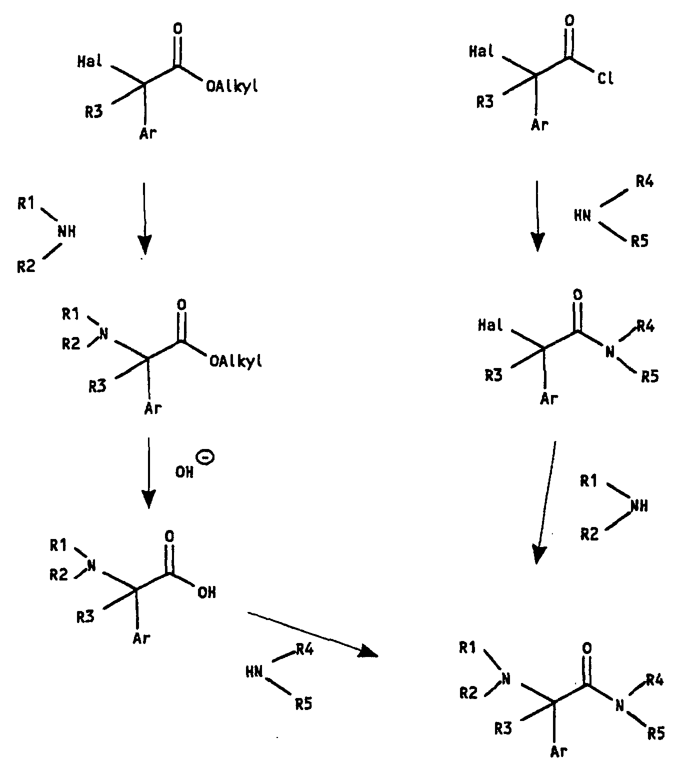

Verfahren A. Die Verknüpfung der Carbonsäure mit dem Amin HN (R⁵)R⁴ kann auf verschiedene Weise erfolgen. Übliche Methoden sind Kupplungsverfahren wie sie in der Peptidchemie angewendet werden. Dabei wird ein Kupplungsreagens wie TBTU, DCCI / HOBt, CDI, etc. in etwa äquivalenter Menge zu den Kupplungspartnern eingesetzt. Geeignete Lösungsmittel sind DMF, THF, CH₂ Cl₂, CHCl₃, Acetonitril oder andere indifferente Lösungsmittel oder deren Gemische. Der geeignete Temperaturbereich liegt zwischen -50°C und + 120°C, bevorzugt zwischen 0°C und 40°C.

Die Carbonsäure kann auch zunächst mittels SOCl₂, SO₂Cl₂, PCl₃, PCl₅ oder PBr₃ oder deren Gemischen nach bekannten Verfahren in das entsprechende Säurehalogenid überführt werden, das anschließend in einem indifferenten Lösungsmittel wie z.B. CH₂Cl₂, THF oder Dioxan bei Temperaturen zwischen - 50°C und +100°C, typischerweise bei 0° bis 20°C mit dem Amin HN (R⁵)R⁴ umgesetzt wird.
Eine weitere Alternative besteht darin, die Carbonsäure nach bekannten Methoden zunächst in den Alkylester, üblicherweise den Methylester zu überführen, der dann in einem indifferenten Lösungsmittel wie z.B. DMF, Dioxan oder THF mit dem Amin HN(R⁵) R⁴ zur Reaktion gebracht wird. Die Reaktionstemperaturen liegen zwischen 20°C und 150°C, typischerweise zwischen 50°C und 120°C. Die Reaktion kann auch in einem Druckbehälter durchgeführt werden.

Verfahren B. Hierbei wird das nach bekannten Vorgehensweisen erhaltene α-Halogen-arylacetamidderivat mit dem Amin R¹(R²)NH unter Abspaltung von Halogen-wasserstoff zur Reaktion gebracht. Zum Abfangen des abgespaltenen (oder auch überschüssigen) Halogenwasserstoffs verwendet man anorganische Basen wie z.B. K₂CO₃, NaHCO₃ oder CaCO₃ oder organische Basen wie z.B. Triethylamin, Hünig-Base, Pyridin oder DMAP, oder man verwendet das Amin R¹(R²)NH im Überschuß. Dabei verwendet man DMF, THF, Dioxan oder andere indifferente Lösungsmittel. Der Temperaturbereich für die Reaktion liegt bei 0° - 100°C, typischerweise zwischen 10° und 80°C.

Verfahren C. Die erfindungsgemäßen Verbindungen in denen R⁵ nicht H ist, können auch wie folgt hergestellt werden: Zunächst synthetisiert man z.B. nach Verfahren A oder B die entsprechende Verbindung in der R⁵ H ist. Anschließend führt man wie folgt eine N-Alkylierung durch, um so Alkyl, Cycloalkyl oder CH₂COOH einzuführen. Die erfindungsgemäße Verbindung worin R⁵ H ist wird mit einer äquivalenten Menge NaH, NaNH₂, KOH, NaOCH₃ oder einer anderen starken Base deprotoniert. Dabei verwendet man wasserfreie, indifferente Lösungsmittel wie z.B. THF, Dioxan oder Diethylether. Anschließend gibt man das entsprechende Alkylierungsmittel in Form des entsprechenden Halogenids, Tosylats oder Mesylats langsam zu. Die Umsetzung wird im Temperaturbereich -50°C bis +100°C durchgeführt, typischerweise zwischen 0°C und +50°C.

### Beispiel 1:

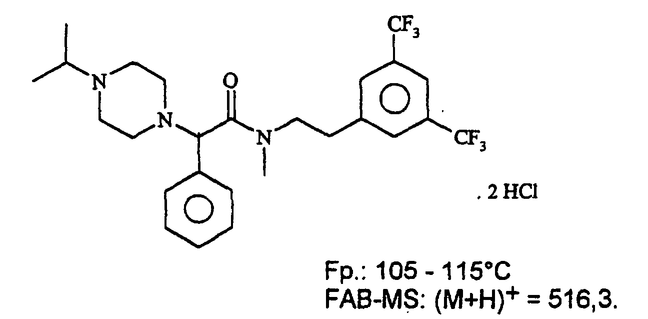

1. Stufe: 0,71 g 1-lsopropylpiperazin wurden in 55 ml wasserfreiem DMF gelöst, mit 0,64 g Na₂CO₃ versetzt, 20 min. bei RT gerührt und dann auf 5°C abgekühlt. Es wurden 1,15 g (R,S)- α-Bromphenylessigsäuremethylester zugegeben und die Suspension über Nacht bei RT gerührt. Der Niederschlag wurde abfiltriert und das Filtrat eingeengt. Der Rückstand wurde in Essigester aufgenommen, 2 x mit 10%iger KHCO₃-Lsg. und 1 x mit gesättigter NaCl-Lsg. extrahiert. Die organische Phase wurde über Na₂SO₄ getrocknet, filtriert und eingeengt, wobei 1,23 g (R,S)-1-lsopropyl-4-(2-phenylessigsäuremethylester)-piperazin als viskoses Öl erhalten wurden.
   Ausbeute: ca. 89%.
2. Stufe: 1,23 g des Produkts aus der 1. Stufe wurden in 10 ml Methanol und 10 ml THF gelöst, mit 10 ml 1 N NaOH versetzt und die Mischung über Nacht bei Raumtemperatur gerührt. Die klare Reaktionslösung wurde durch Zugabe von 10 ml 1 N HCl neutralisiert, zur Trockne eingeengt, der Rückstand mit DMF behandelt und der Feststoff abgesaugt. Das Filtrat wurde eingeengt und der Rückstand mit Ether verrieben, der Feststoff abgesaugt und im Exsikkator getrocknet. So wurden 1,1 g (R,S)-1-i-Propyl-4-(2-phenylessigsäure)-piperazin als weisse Festsubstanz erhalten. Ausbeute: 92%.
3. Stufe: 0,37 g des Produkts aus der 2.Stufe und 0,42 g N-Methyl-3,5-bis-(trifluormethyl)-phenylethylamin wurden in 14 ml DMF gelöst und durch Zugabe von ca. 0,4 ml TEA auf pH 8,5 eingestellt. Es wurde mit 0,48 g TBTU versetzt und bei Raumtemperatur über Nacht gerührt. Die klare Reaktionslösung wurde unter Vakuum eingeengt, der Rückstand mit NaHCO₃-Lösung verrührt und 2 x mit Essigester extrahiert. Die vereinigten organischen Phasen wurden filtriert und das Filtrat eingeengt. Der Rückstand wurde mit CH₂Cl₂/MeOH (9:1) als Eluens über Kieselgel chromatographiert. Die erhaltenen einheitlichen Fraktionen wurden eingeengt, in wenig MeOH gelöst, mit etherischer HCI angesäuert und wiederum eingeengt. Der Rückstand wurde mit Ether verrieben und im Exsikkator getrocknet. Es wurden 0,58 g (R,S)-1-i-Propyl-4-[2-phenylessigsäure-N-methyl-N-(3,5-bistrifluormethyl-phenylethyl)-amid Dihydrochlorid als weiße Festsubstanz erhalten.
   Ausbeute: 75%.

Analog können die anderen Verbindungen dieser Erfindung hergestellt werden, zum Beispiel die folgenden:

### Beispiel 2:

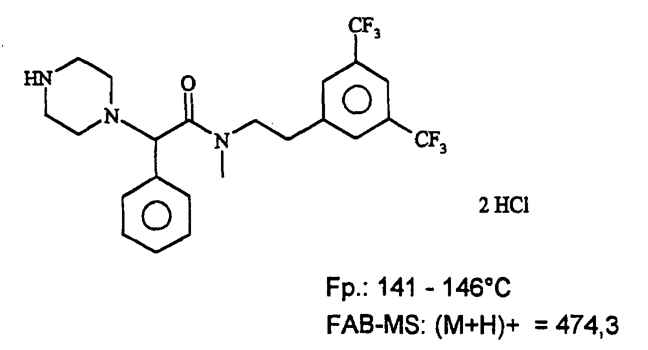

### Beispiel 3:

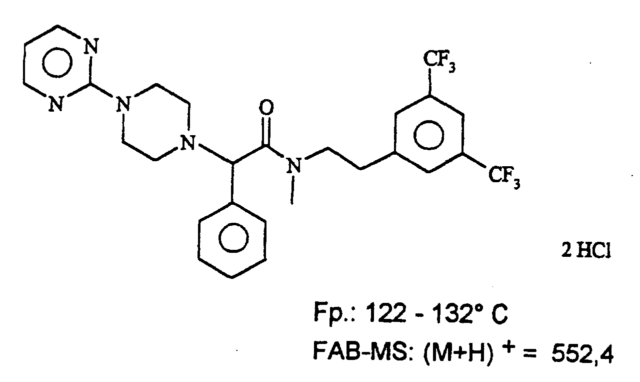

### Beispiel 4:

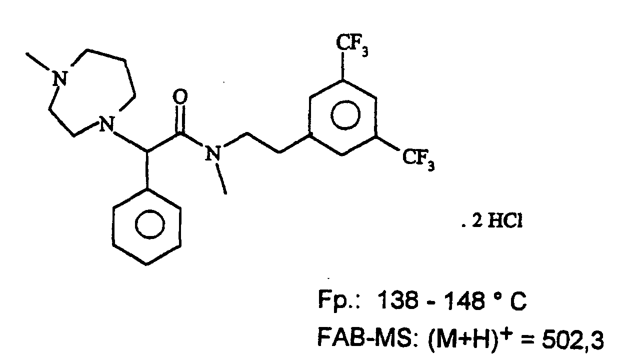

### Beispiel 5:

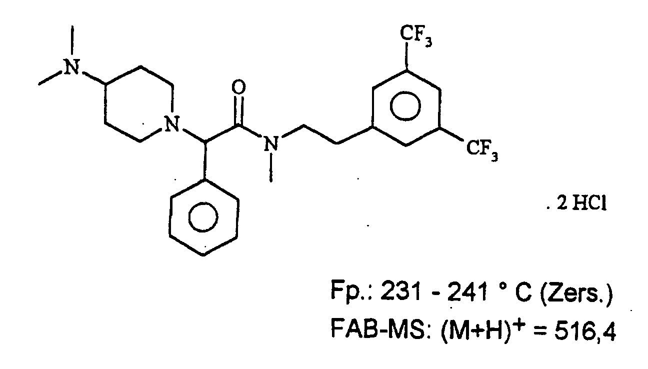

### Beispiel 6:

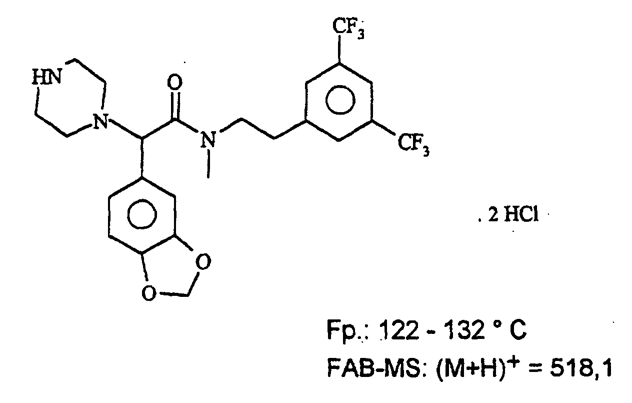

### Beispiel 7:

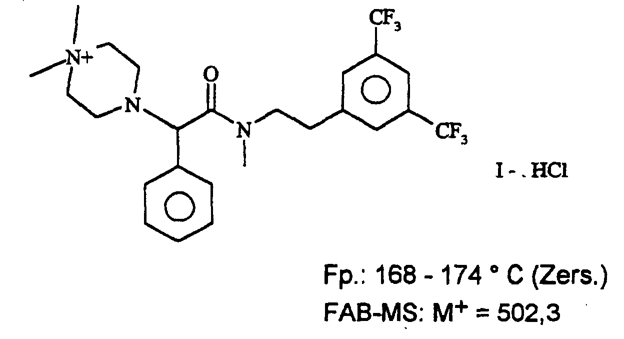

### Beispiel 8:

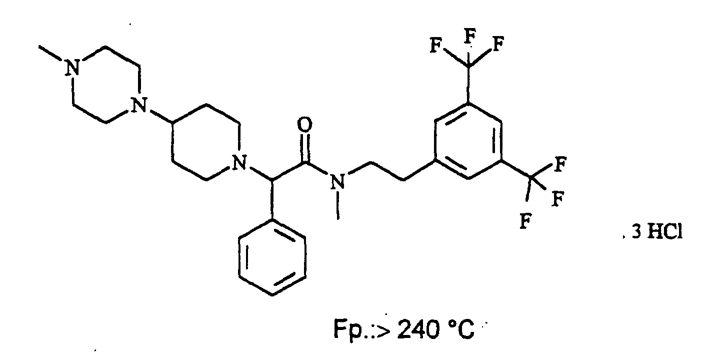

### Beispiel 9:

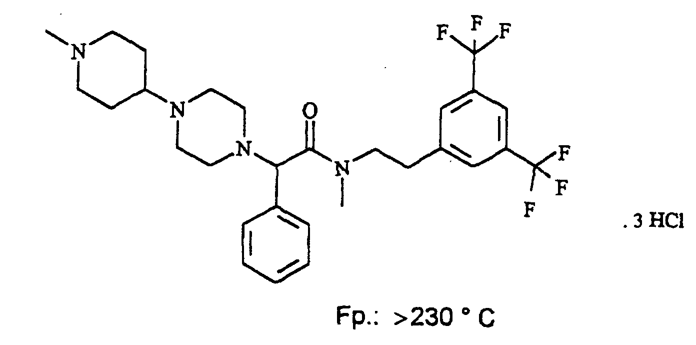

### Beispiel 11:

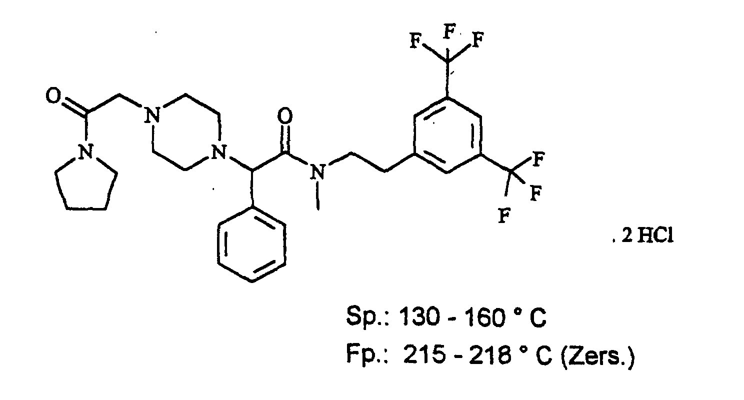

### Beispiel 12:

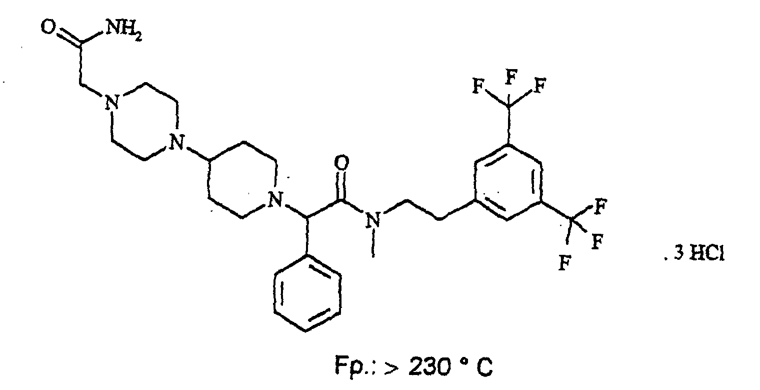

### Beispiel 13:

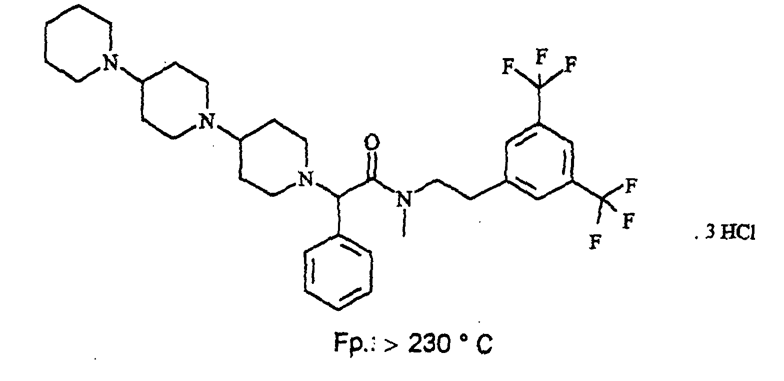

### Beispiel 15:

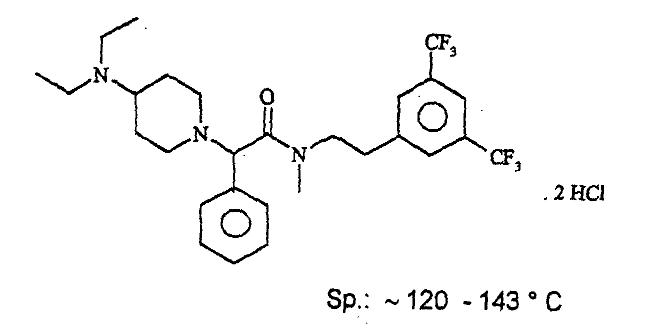

### Beispiel 16:

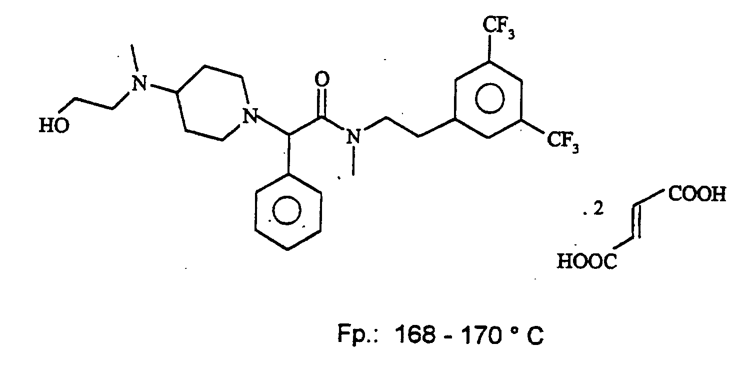

### Beispiel 17:

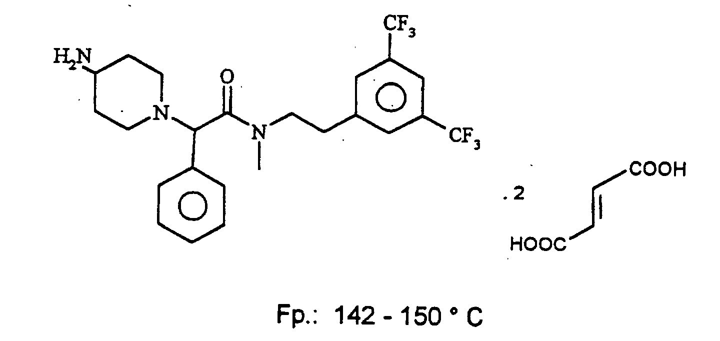

### Beispiel 18:

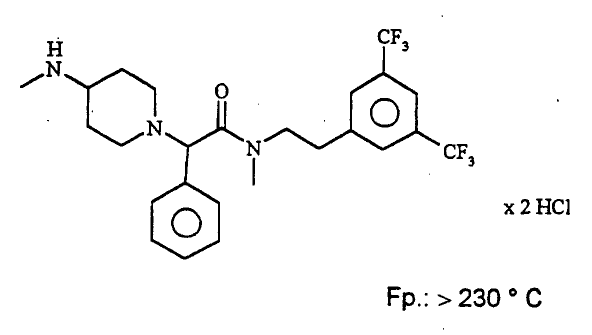

### Beispiel 19:

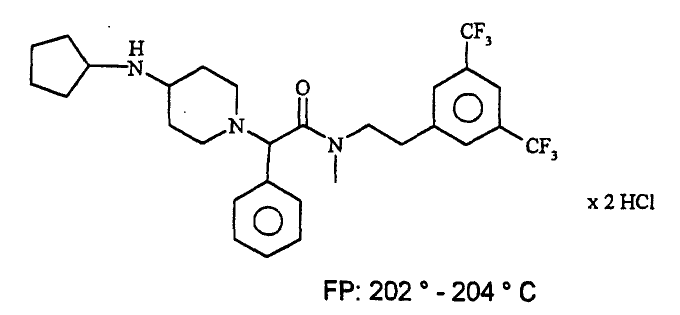

### Beispiel 20:

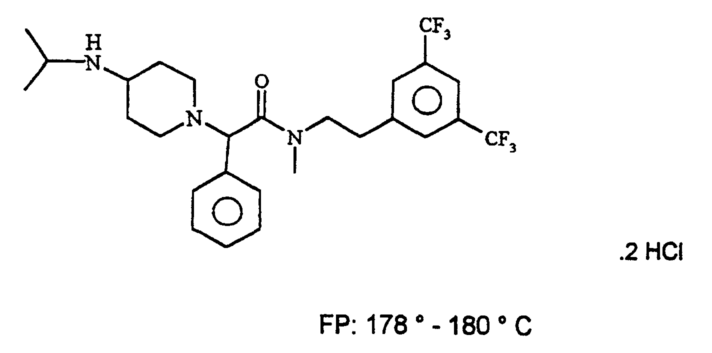

### Beispiel 22:

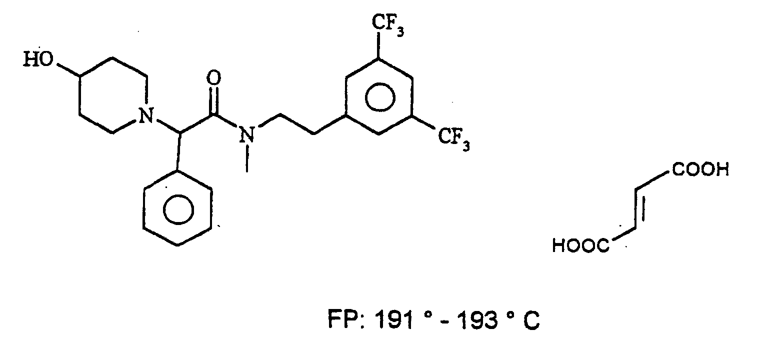

### Beispiel 23:

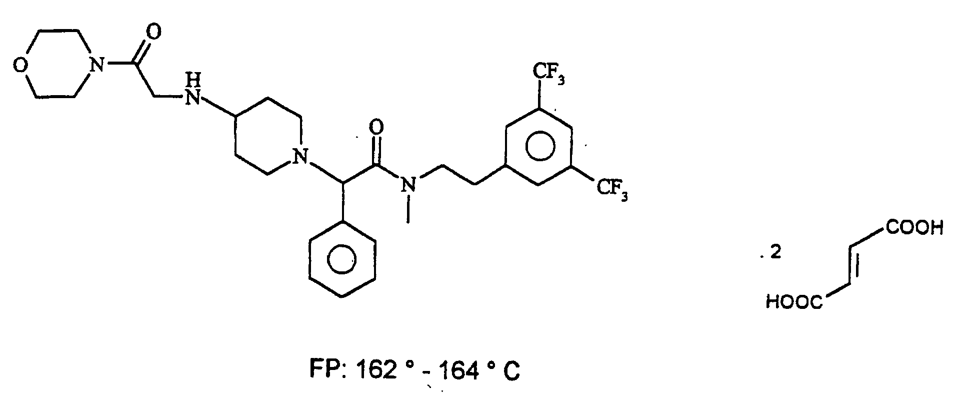

### Beispiel 24:

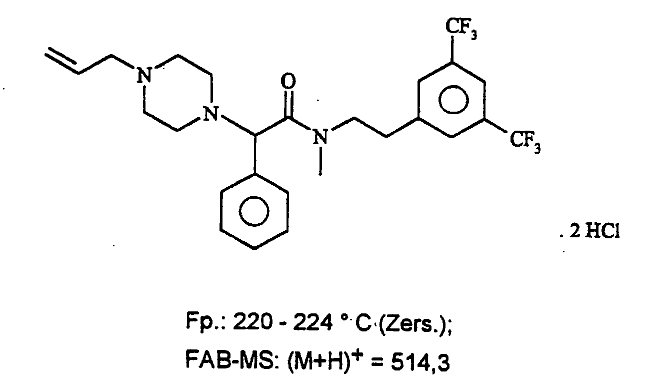

### Beispiel 25:

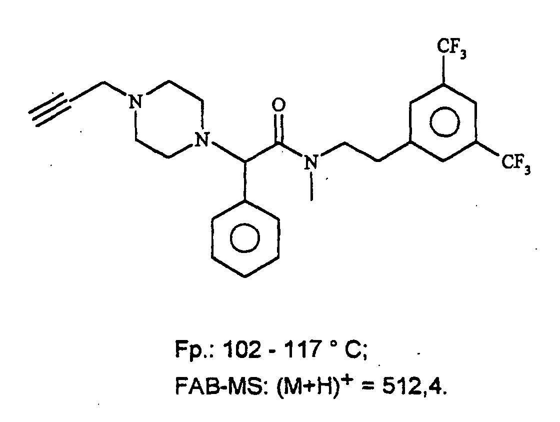

### Beispiel 26:

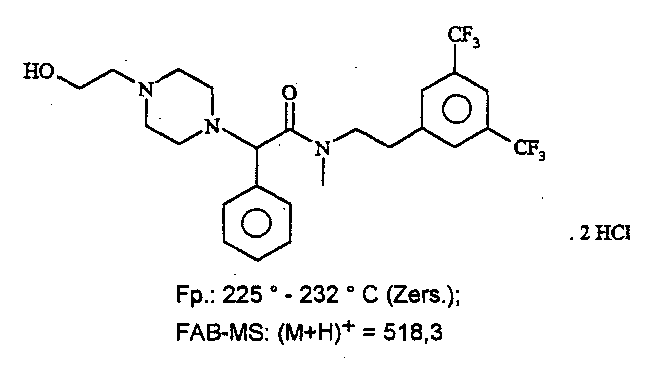

### Beispiel 27:

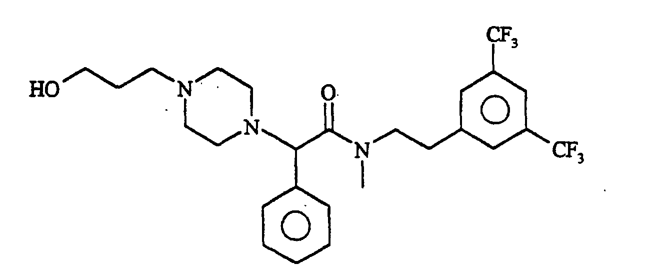

### Beispiel 28:

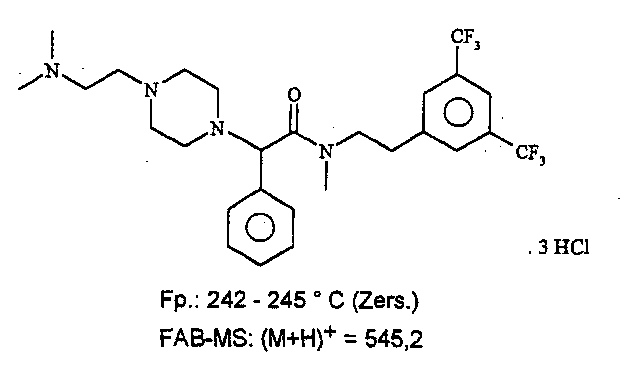

### Beispiel 29:

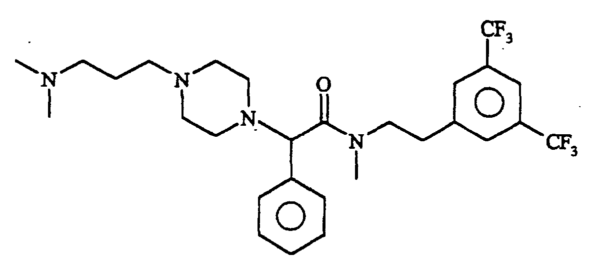

### Beispiel 30:

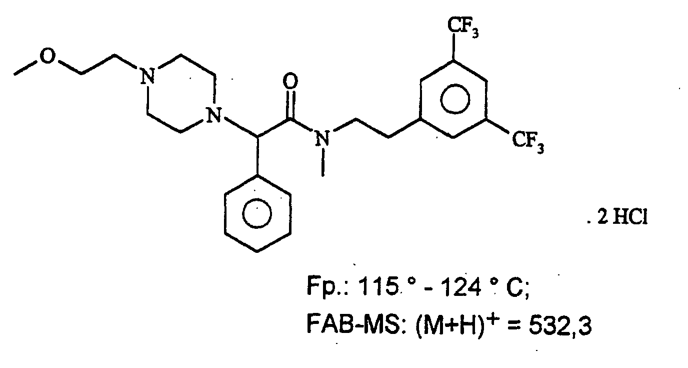

### Beispiel 31:

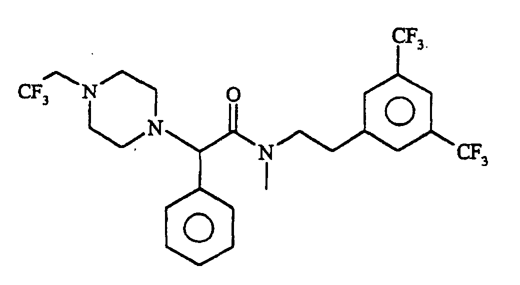

### Beispiel 32:

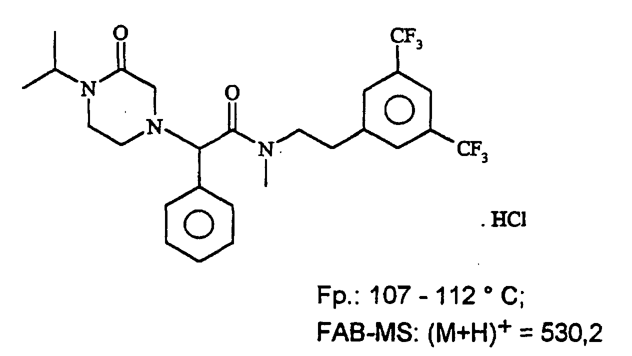

### Beispiel 33:

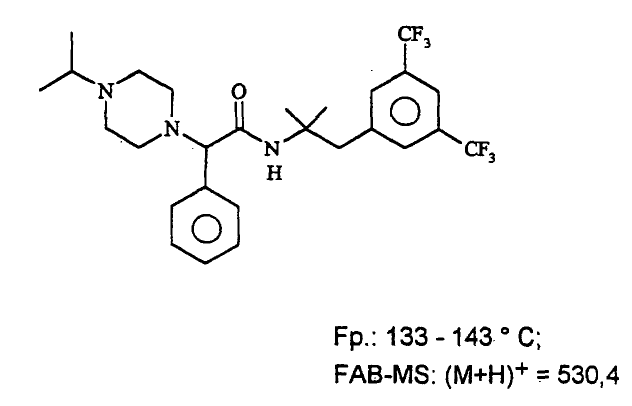

### Beispiel 34:

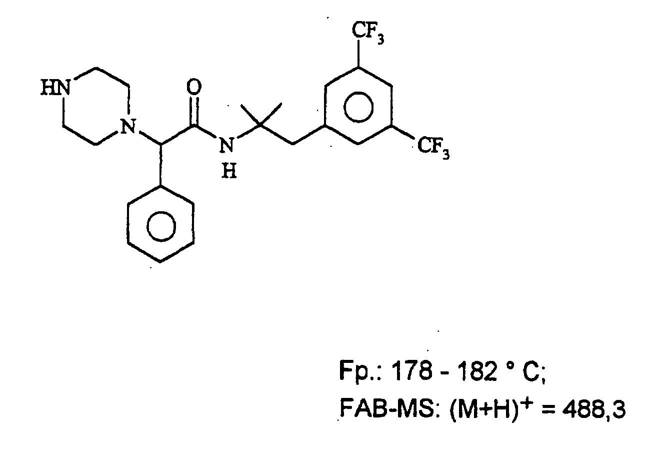

### Beispiel 35:

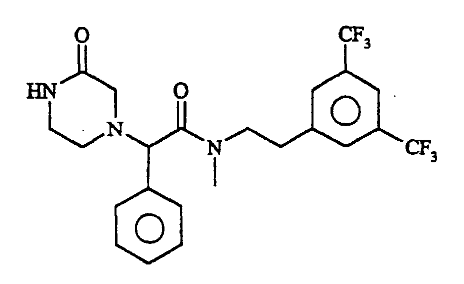

### Beispiel 36:

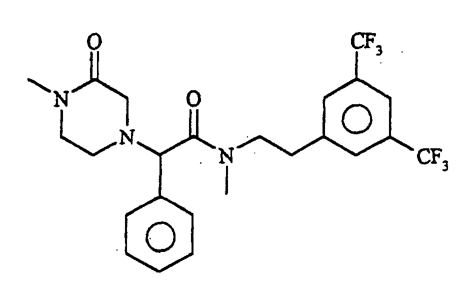

### Beispiel 37:

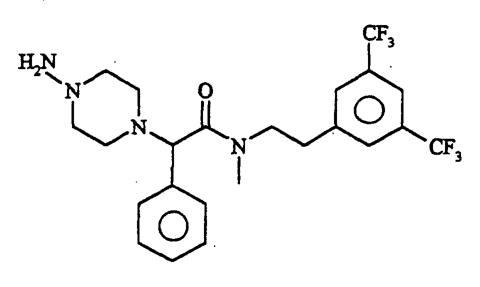

### Beispiel 38:

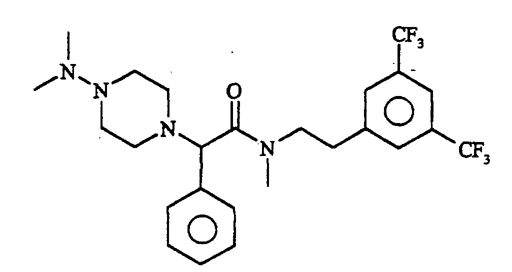

### Beispiel 39:

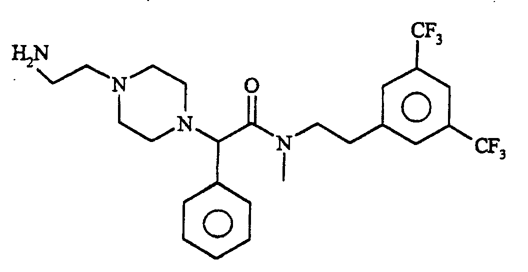

### Beispiel 40:

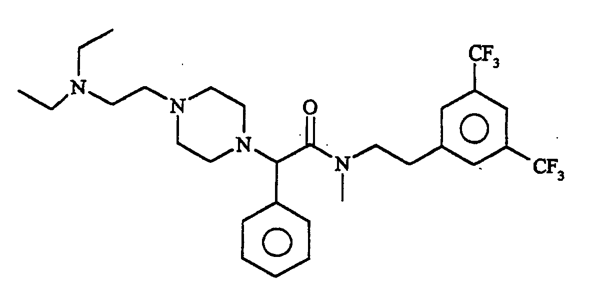

### Beispiel 41:

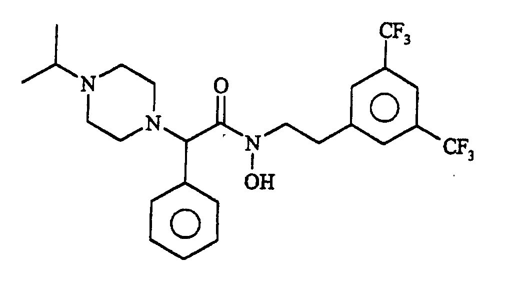

### Beispiel 42:

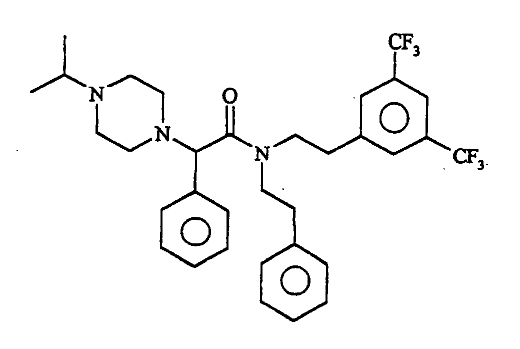

### Beispiel 43:

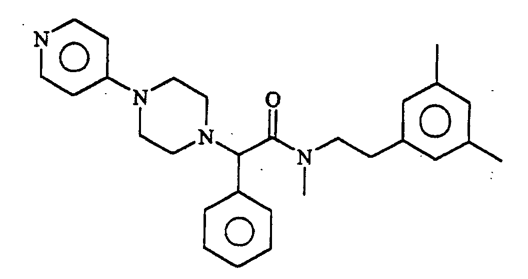

### Beispiel 44:

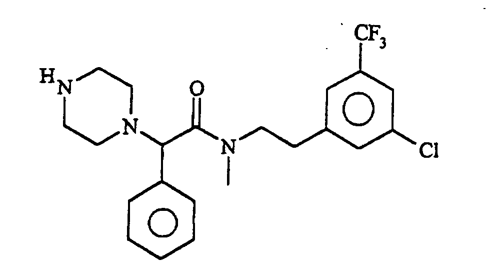

### Beispiel 45:

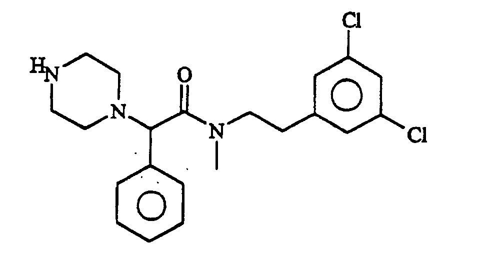

### Beispiel 46:

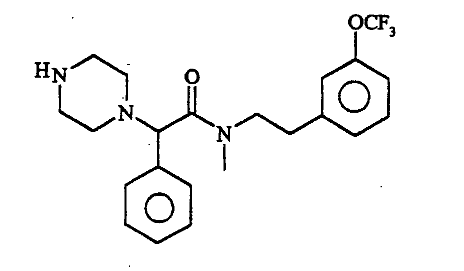

### Beispiel 47:

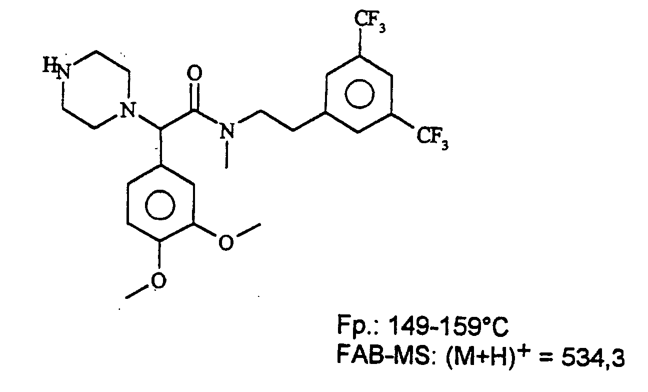

### Beispiel 48:

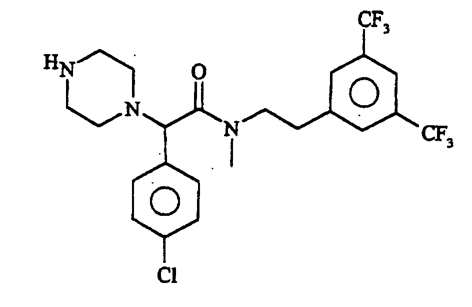

### Beispiel 49:

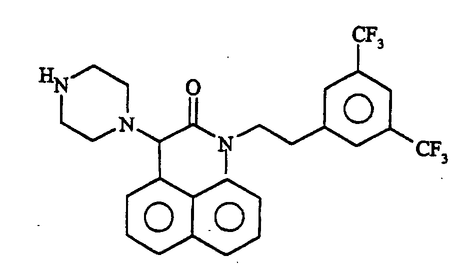

### Beispiel 50:

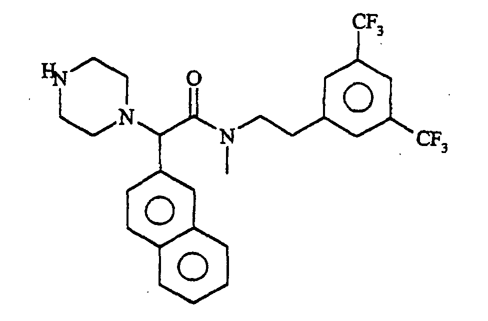

### Beispiel 51:

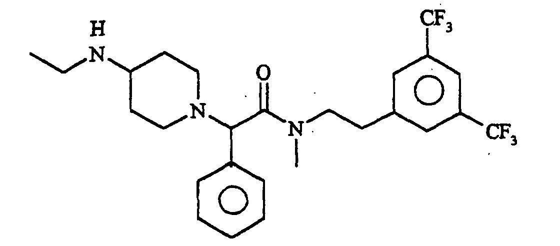

### Beispiel 53:

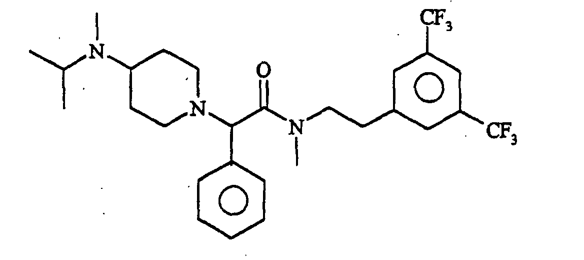

### Beispiel 54:

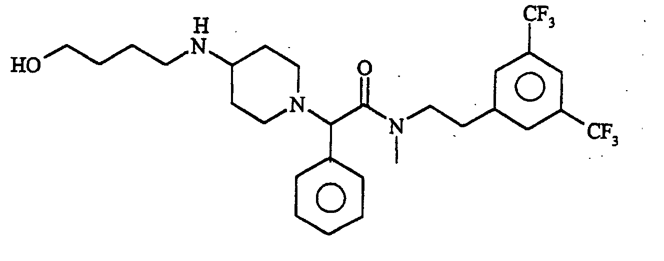

### Beispiel 55:

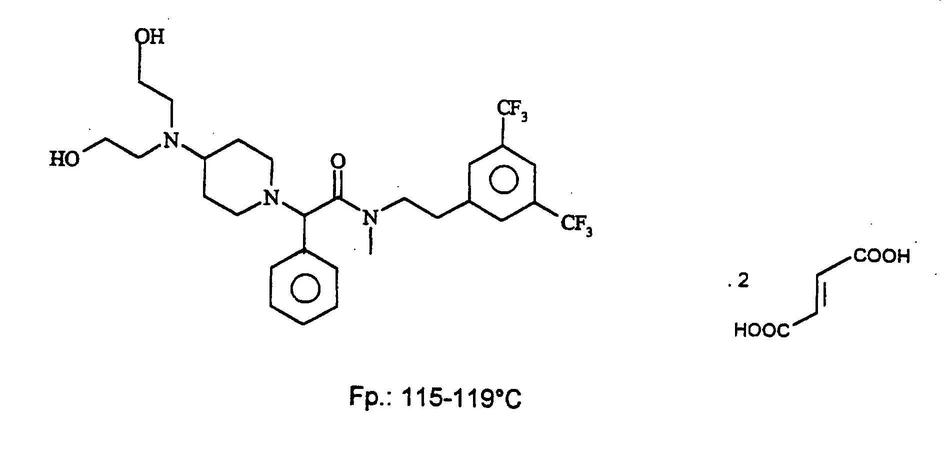

### Beispiel 56:

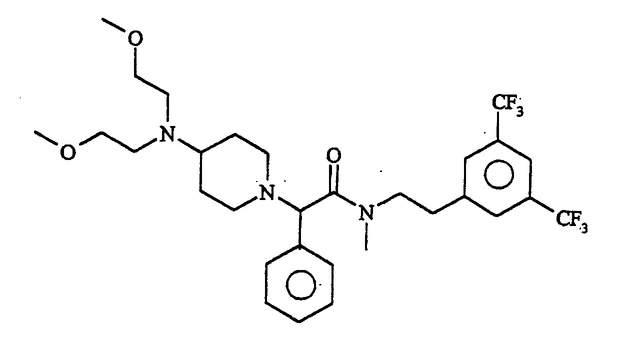

### Beispiel 57:

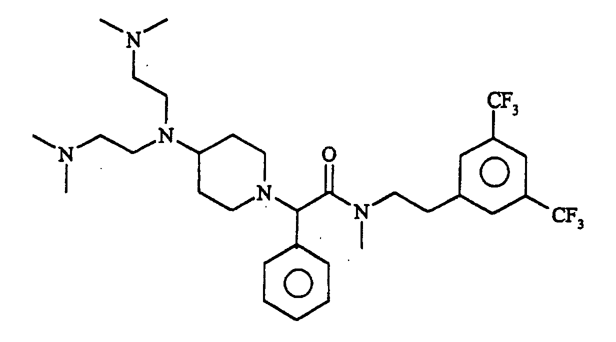

### Beispiel 58:

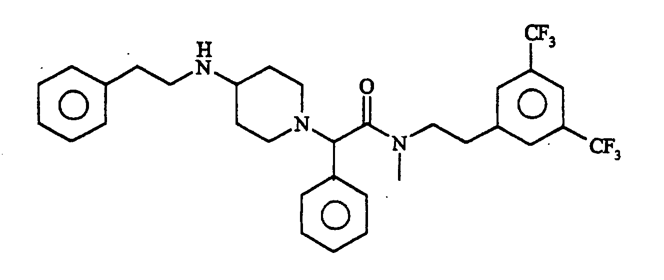

### Beispiel 59:

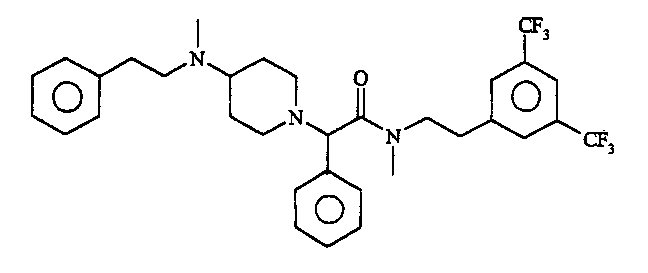

### Beispiel 60:

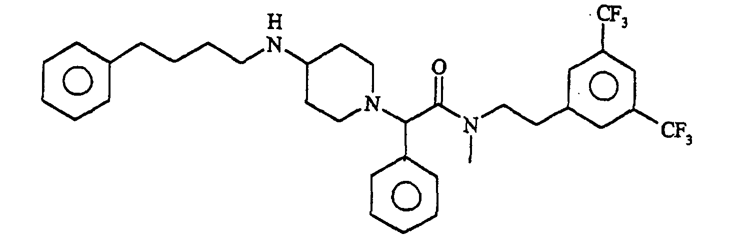

### Beispiel 61:

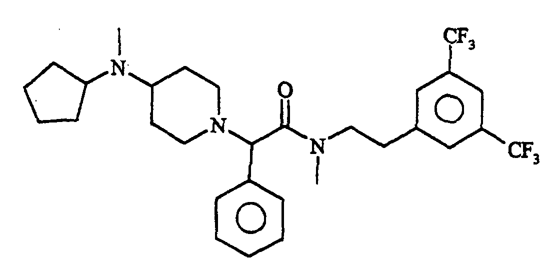

### Beispiel 63:

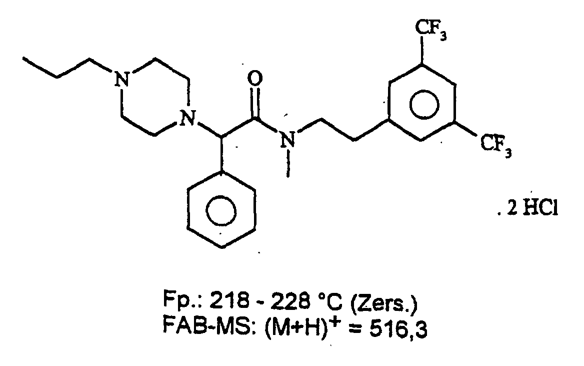

### Beispiel 64:

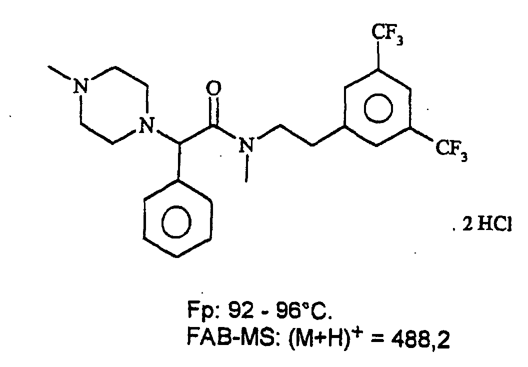

### Beispiel 65

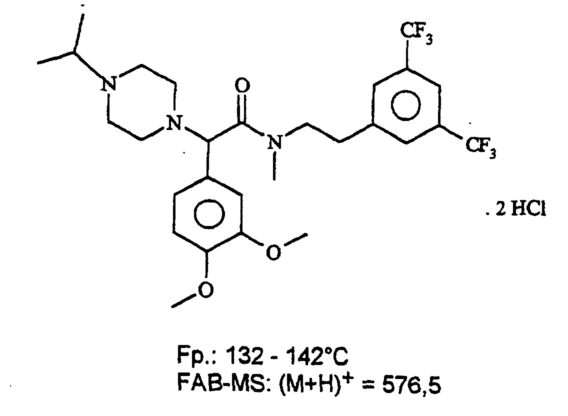

### Beispiel 66:

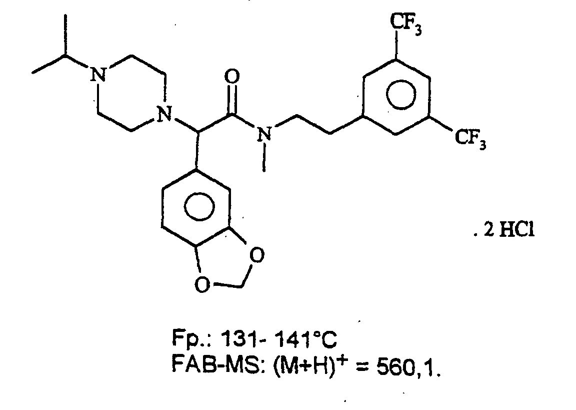

### Beispiel 67:

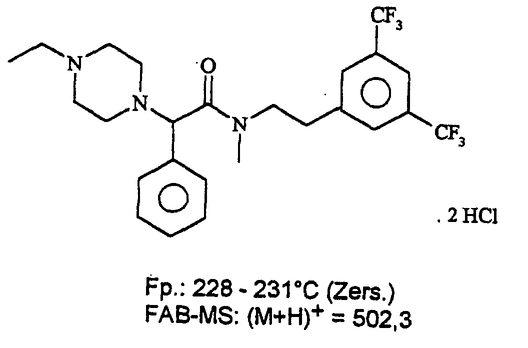

### Beispiel 68:

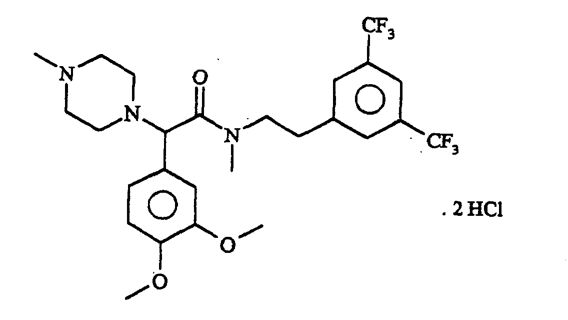

### Beispiel 69:

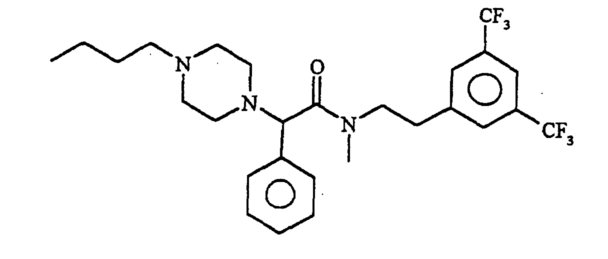

### Beispiel 70:

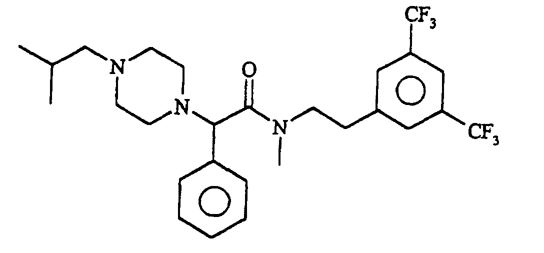

### Beispiel 71:

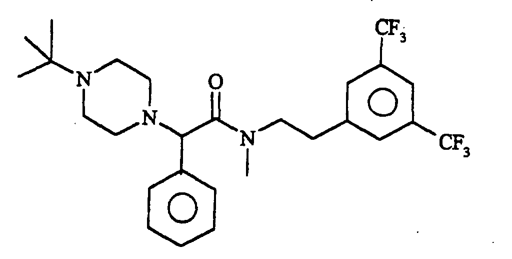

### Beispiel 72:

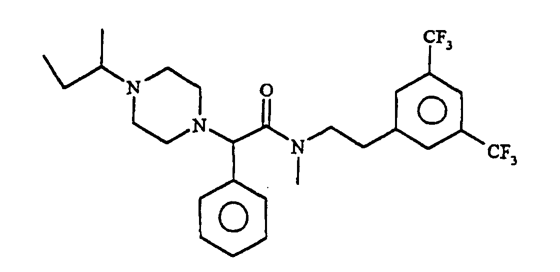

### Beispiel 73:

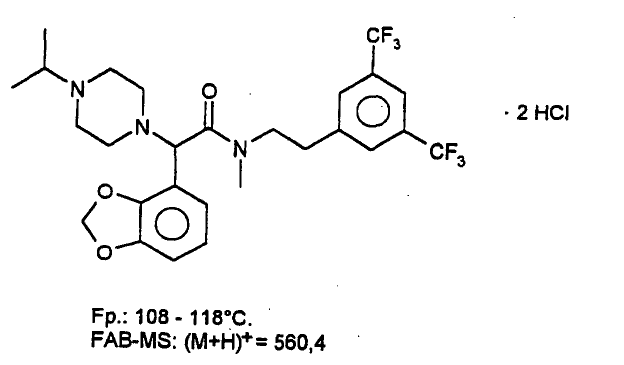

### Beispiel 74:

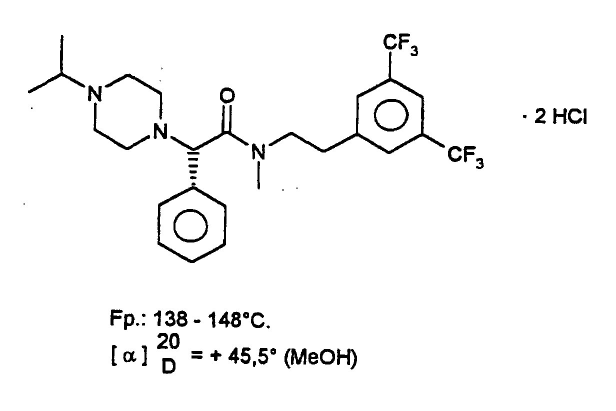

### Beispiel 75:

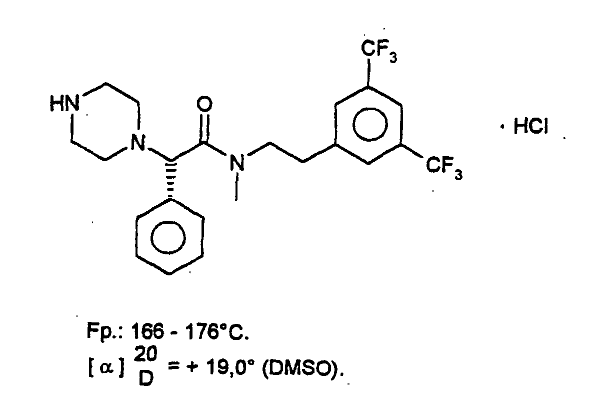

### Beispiel 76:

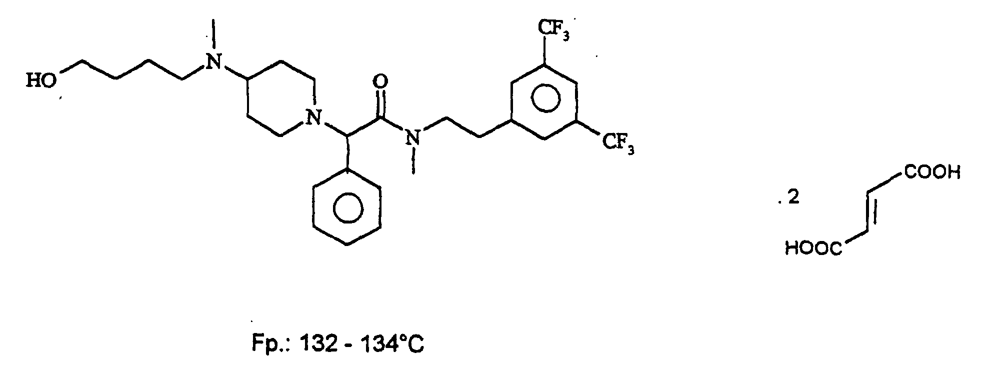

von diesen Verbindungen sind die Verbindungen der Beispiele 1 und 8 bevorzugt.

In der Darstellung der obigen Formeln wurde die Art verwendet, bei der CH₃-Gruppen nicht ausgeschrieben werden.
Verbindung 1 zum Beispiel enthält als Gruppe R⁵ eine Methylgruppe.

### Pharmazeutische Zubereitungen:

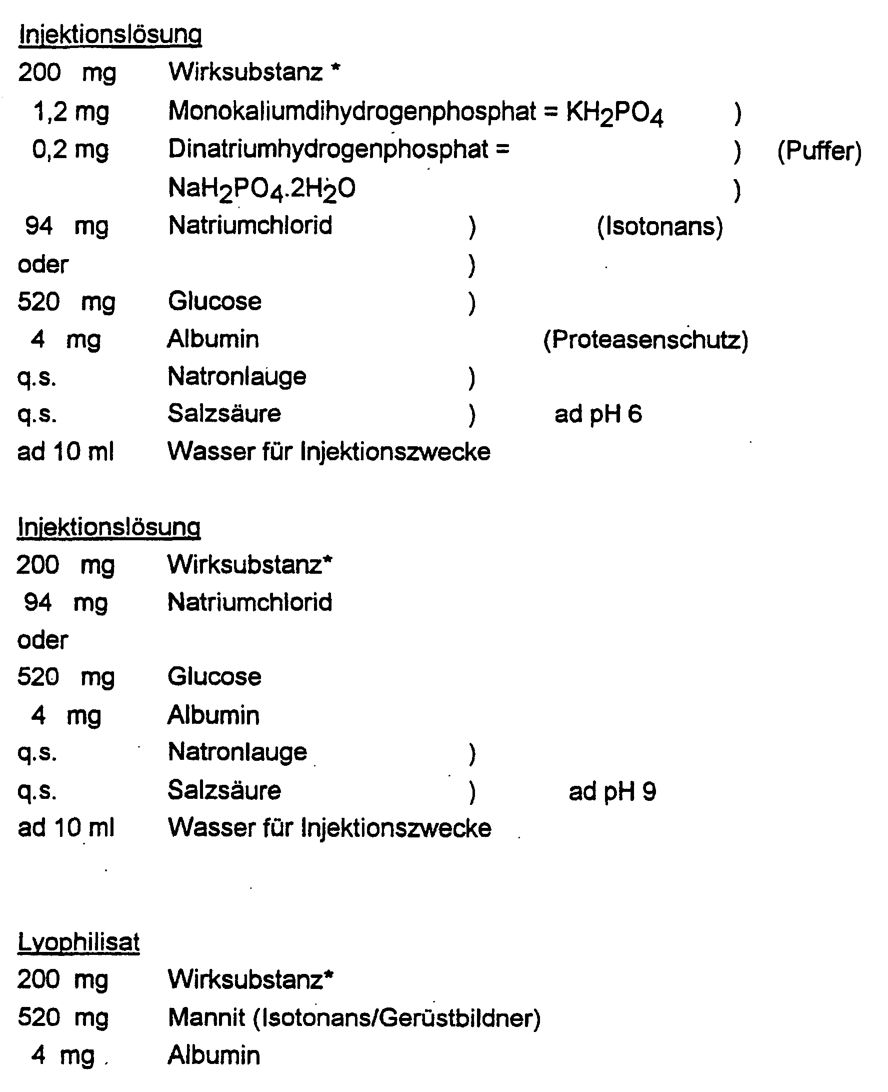

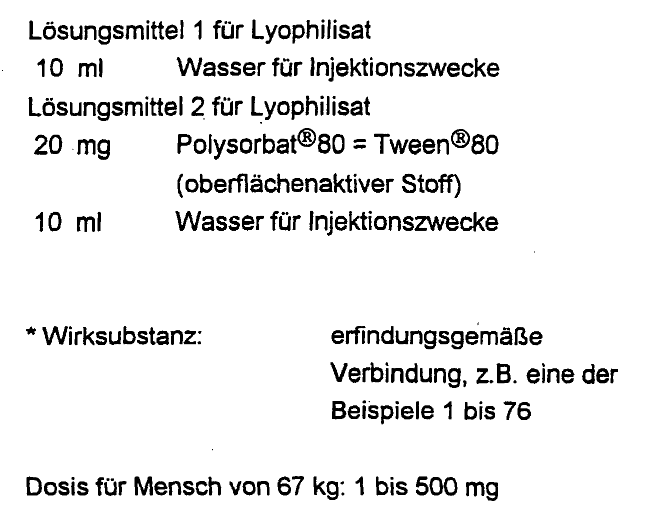

## Patentansprüche

1. Arylglycinamidderivate der allgemeinen Formel l 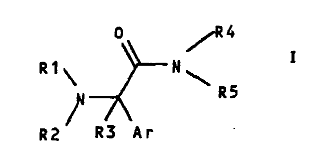 oder deren pharmazeutisch annehmbare Salze,
worin
Ar unsubstituiertes oder 1- bis 5-fach substituiertes Phenyl, oder unsubstituiertes oder 1- oder 2-fach substituiertes Naphthyl ist, [wobei die Substituenten des Phenyl und Naphthyl unabhängig voneinander Halogen (F, Cl, Br, J), (C₁-C₄)Alkyl, O-(C₁-C₄)Alkyl, CF₃, OCF₃ oder NR¹²R¹³ (worin R¹² und R¹³ unabhängig voneinander H, Methyl oder Acetyl sind) sind] oder Ar durch -O-CH₂-O- oder -O-(CH2)2-O- substituiertes Phenyl ist;
R¹ und R² zusammen mit dem N, an den sie gebunden sind, einen Ring der Formel 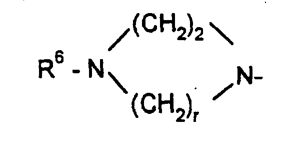 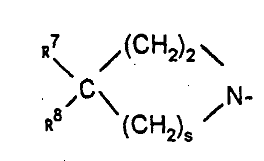 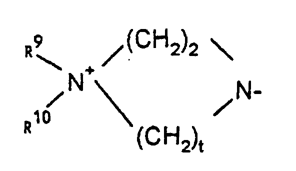 oder 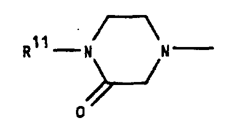 bilden, worin
r, s und t 2 oder 3 sind;
R⁶
H,
(C₁-C₅)Alkyl,
(C₃-C₅)Alkenyl,
Propinyl,
Hydroxy(C₂-C₄)alkyl,
Methoxy(C₂-C₄)alkyl,
Di(C₁-C₃)alkylamino(C₂-C₄)alkyl,
Amino(C₂-C₄)alkyl,
Amino,
Di(C₁-C₃)alkylamino,
Monofluor- bis Perfluor(C₁-C₂)alkyl,
N-Methylpiperidinyl,
Pyridyl,
Pyrimidinyl,
Pyrazinyl,
Pyridazinyl
oder die Gruppe -CH₂-C(O)NR¹⁴R¹⁵ bedeutet,
worin
R¹⁴ H oder (C₁-C₄)Alkyl ist und
R¹⁵ H, (C₁-C₄)Alkyl, (C₃-C₆)Cycloalkyl, Hydroxy(C₂-C₄)alkyl, Alkoxy(C₂-C₃)alkyl, Phenyl(C₁-C₄)alkyl, oder R¹⁴ und R¹⁵ zusammen mit dem N an den sie gebunden sind einen Ring (1-Pyrrolidinyl, Piperidino, Morpholino oder 1-Methylpiperazin-4-yl) bilden;
R⁷ eine der Bedeutungen (a) bis (d) hat,
(a) Hydroxy
(b) 4-Piperidinopiperidyl,
(c) 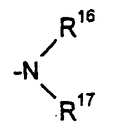 worin R¹⁶ und R¹⁷ unabhängig voneinander
H,
(C₁-C₄)Alkyl,
(C₃-C₆)Cycloalkyl,
Hydroxy(C₂-C₄)alkyl,
(C₁-C₃)Alkoxy(C₂-C₄)alkyl,
Phenyl(C₁-C₄)alkyl oder
Di(C₁-C₃)alkylamino(C₂-C₄)alkyl sind,
oder wenn R¹⁶ H oder (C₁-C₄)Alkyl ist,
R¹⁷ auch -CH₂C(O)NR¹⁸R¹⁹ sein kann, worin R¹⁸ und R¹⁹
wie obige R¹⁴ und R¹⁵ definiert sind;
(d) 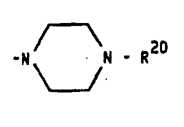 worin R²⁰
H
(C₁-C₄)Alkyl,
(C₄-C₆)Cycloalkyl oder
-CH₂C(O)NR²¹R²² ist,
worin R²¹ und R²² wie obige R¹⁴ und R¹⁵ definiert sind;
R⁸ H ist
R⁹ und R¹⁰ unabhängig voneinander
(C₁-C₄)Alkyl sind;
R¹¹
H,
(C₁-C₅)Alkyl,
(C₃-C₅)Alkenyl,
Propinyl,
Hydroxy(C₂-C₄)alkyl,
Methoxy(C₂-C₃)alkyl,
Di(C₁-C₃)alkylamino(C₂-C₃)alkyl,
Amino(C₂-C₃)alkyl,
Amino,
Di(C₁-C₃)alkylamino,
Monofluor- bis Perfluor(C₁-C₂)alkyl,
N-Methylpiperidinyl,
Pyridyl,
Pyrimidinyl,
Pyrazinyl,
Pyridazinyl
oder die Gruppe -CH₂-C(O)NR²³R²⁴ bedeutet,
worin R²³ und R²⁴ wie obige R¹⁴ und R¹⁵ definiert sind;
R³ H, (C₁-C₄)Alkyl, unsubstituiertes oder 1-3fach substituiertes Phenyl ist, worin die Substituenten unabhängig voneinander Halogen (F, Cl, Br, J), (C₁-C₄)Alkyl, O-(C₁-C₄)Alkyl, CF₃, OCF₃ oder NR²⁵R²⁶ (worin R²⁵ und R²⁶ unabhängig voneinander H, Methyl oder Acetyl sind) sind;
R4 Phenyl(C₁-C₄)alkyl oder Naphthyl(C₁-C₄)alkyl bedeutet, worin Phenyl durch 1 bis 3 Substituenten substituiert sein kann, worin die Substituenten unabhängig voneinander Halogen (F, Cl, Br, J), (C₁-C₄)Alkyl, O-(C₁-C₄)Alkyl, CF₃, OCF₃ oder NR²⁷R²⁸ (worin R²⁷ und R²⁸ unabhängig voneinander H, Methyl oder Acetyl sind) sind;
und
R5 H, (C₁-C₄)Alkyl, (C₃-C₆)Cycloalkyl, CH₂COOH, CH₂C(O)NH₂, OH oder Phenyl(C₁-C₄)alkyl bedeutet.

2. Verbindung nach Anspruch 1,
worin
Ar unsubstituiertes oder 1- oder 2-fach substituiertes Phenyl, oder unsubstituiertes Naphthyl ist, oder Ar durch -O-CH₂-O- oder -O-(CH₂)₂-O- substituiertes Phenyl ist;
R¹ und R² zusammen mit dem N, an den sie gebunden sind, einen Ring der Formel 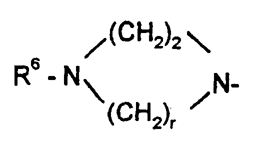 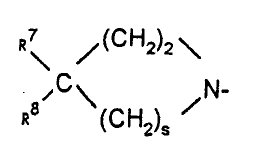 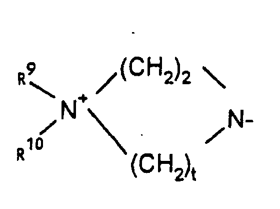 oder 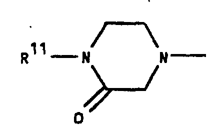 bilden, worin
r 2 oder 3 ist und
s und t 2 sind;
R⁶, R⁷, R⁸, R⁹, R¹⁰ und R¹¹ wie in Anspruch 1 definiert sind;
R³ H oder (C₁-C₄)Alkyl ist,
R⁴ Phenyl(C₁-C₄)alkyl oder Naphthyl(C₁-C₄)alkyl bedeutet, worin Phenyl durch 1 oder 2 Substituenten substituiert sein kann, worin die Substituenten unabhängig voneinander Halogen (F, Cl, Br, J), (C₁-C₄)Alkyl, O-(C₁-C₄)Alkyl, CF₃ oder OCF₃ sind;
und
R⁵ H, (C₁-C₄)Alkyl, (C₃-C₆)Cycloalkyl, OH oder Phenyl(C₁-C₄)alkyl bedeutet.

3. Verbindung nach Anspruch 1 oder 2, worin
Ar unsubstituiertes oder 1- oder 2-fach substituiertes Phenyl, oder unsubstituiertes Naphthyl ist, [wobei die Substituenten des Phenyl unabhängig voneinander Halogen (F, Cl, Br, J), Methyl, Methoxy, CF₃ oder OCF₃ sind] oder Ar durch -O-CH₂-O- oder -O-(CH₂)₂-O- substituiertes Phenyl ist.

4. Verbindung nach Anspruch 3, worin Ar Phenyl, Naphthyl, in Position 3 und/oder 4 durch Methoxy oder Halogen substituiertes Phenyl ist oder Phenyl ist, dessen Positionen 2 und 3, oder 3 und 4 durch -O-CH₂-O- verbunden sind.

5. Verbindung nach Anspruch 4, worin Ar Phenyl,
in Positionen 3 und 4 durch Methoxy substituiertes Phenyl ist oder Phenyl ist, dessen Positionen 3 und 4 oder 2 und 3 durch -O-CH₂-O- verbunden sind.

6. Verbindung nach einem der Ansprüche 1 bis 5, worin in dem Ring 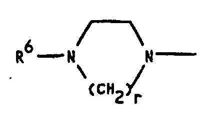 r 2 oder 3 ist, und
R6
H,
(C₁-C₅)Alkyl,
(C₃-C₅)Alkenyl,
Propinyl,
Hydroxy(C₂-C₄)alkyl,
Methoxy(C₂-C₄)alkyl,
Di(C₁-C₃)alkylamino(C₂-C₄)alkyl,
Amino(C₂-C₄)alkyl,
Amino,
Di(C₁-C₃)alkylamino,
Monofluor- bis Perfluor(C₁-C₂)alkyl,
N-Methylpiperidinyl,
Pyridyl,
Pyrimidinyl,
oder 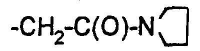 ist.

7. Verbindung nach Anspruch 6, worin r 3 ist und R⁶ Methyl.

8. Verbindung nach Anspruch 6, worin r 2 ist und
R⁶
H,
(C₁-C₄)Alkyl,
Propenyl,
Propinyl,
Hydroxy(C₂-C₃)alkyl,
Methoxyethyl,
Di(C₁-C₂)alkylamino(C₂-C₃)alkyl,
Aminoethyl,
Amino,
Dimethylamino,
CH₂ CF₃,
N-Methylpiperidinyl,
Pyridyl,
Pyrimidinyl,
oder 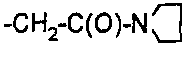 ist.

9. Verbindung nach Anspruch 8, worin
r 2 ist und
R⁶ H, (C₁-C₃)Alkyl, Allyl, 2-Propinyl, -CH₂CH₂OCH₃, -CH₂CH₂N(CH₃)₂, N-Methylpiperidinyl, 2-Pyrimidinyl
oder 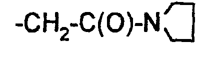 ist.

10. Verbindung nach Anspruch 9, worin
r 2 ist und
R⁶ H, CH₃, C₃H₇, CH(CH₃)₂,
CH₂CH₂OCH₃ oder CH₂CH₂N(CH₃)₂.

11. Verbindung nach einem der Ansprüche 1 bis 5, worin
R¹ und R² zusammen mit dem N, an den sie gebunden sind, den Ring 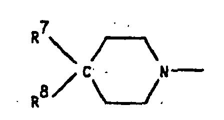 bilden,
worin R⁸ H ist und
R⁷
OH 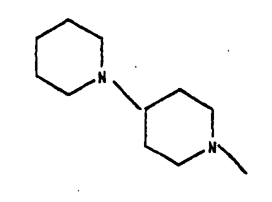 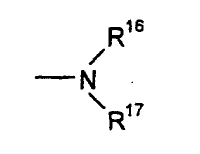 worin R¹⁶ und R¹⁷ unabhängig voneinander sind:
H
(C₁-C₃)Alkyl 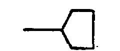 (CH₂)ₙ OH worin n 2, 3 oder 4 ist
(CH₂)₂ OCH₃
-(CH₂)ₙPh worin n 2 oder 4 ist
(CH₂)₂ N(CH₃)₂ 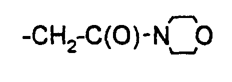 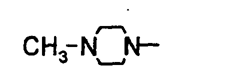 oder

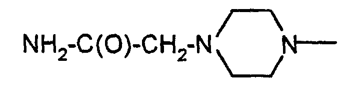

12. Verbindung nach Anspruch 11, worin
R¹⁶ und R¹⁷ beide CH₃ oder C₂H₅ sind oder
R¹⁶ H oder CH₃ ist und R¹⁷
(C₁-C₃)Alkyl 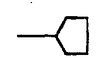 (CH₂)₂OH
(CH₂)₄OH oder

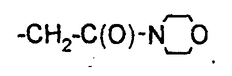

13. Verbindung nach Anspruch 11, worin
R⁷ 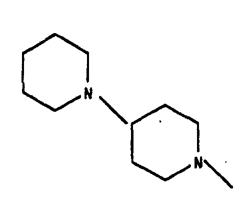 N(CH₃)₂ 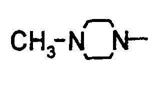 oder 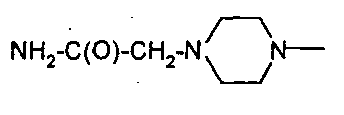 ist.

14. Verbindung nach Anspruch 11, worin
R¹ und R² zusammen mit dem N, an den sie gebunden sind, den Ring 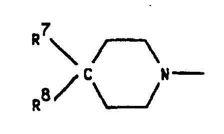 bilden,
worin
(a) R⁸ H ist und
R⁷ 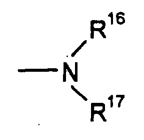 worin
R¹⁶ und R¹⁷ beide CH₃, C₂H₅ oder
CH₂CH₂OH sind oder
R¹⁶ H oder CH₃ ist und R¹⁷
(C₁-C₃)Alkyl 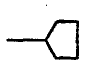 (CH₂)₂OH oder
(CH₂)₄OH
oder
(b) R⁸ H ist und
R⁷

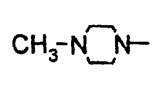

15. Verbindung nach einem der Ansprüche 1 bis 5, worin
R¹ und R² zusammen mit dem N, an den sie gebunden sind, den Ring 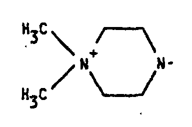 bilden.

16. Verbindung nach einem der Ansprüche 1 bis 5, worin
R¹ und R² zusammen mit dem N, an den sie gebunden sind, den Ring 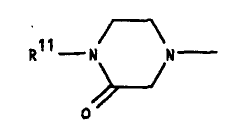 bilden, worin R¹¹ H oder(C₁-C₃)Alkyl ist

17. Verbindung nach Anspruch 16, worin
R¹¹ -CH(CH₃)₂ ist.

18. Verbindung nach einem der Ansprüche 1 bis 17, worin R³ H ist.

19. Verbindung nach einem der Ansprüche 1 bis 18, worin
R⁴ Phenyl(C₁-C₄)alkyl bedeutet, worin Phenyl durch 1 oder 2 Substituenten substituiert sein kann, worin die Substituenten unabhängig voneinander Halogen (F, Cl, Br, J), (C₁-C₄)Alkyl, O-(C₁-C₄)Alkyl, CF₃ oder OCF₃ sind;
und
R⁵ H, (C₁-C₄)Alkyl, (C₃-C₆)Cycloalkyl, -OH oder Phenyl(C₁-C₄)alkyl bedeutet.

20. Verbindung nach Anspruch 19, worin
R⁴ Phenyl(C₂-C₄)alkyl bedeutet, worin die Substituenten in den Positionen 3 und/oder 5 des Phenylringes sind und
R⁵ H, Methyl, OH oder Phenethyl ist.

21. Verbindung nach Anspruch 20, worin
R4 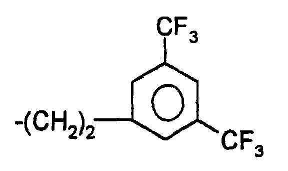 ist und R⁵ Methyl ist.

22. Verfahren zur Herstellung einer Verbindung der allgemeinen Formel 1 nach einem der Ansprüche 1 bis 21, **dadurch gekennzeichnet, daß** man
a) eine Säure 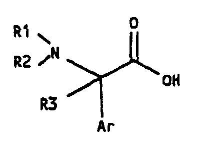 oder deren Halogenid oder Alkylester mit einem Amin 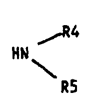 umsetzt;
b) ein α-Halogenarylacetamid 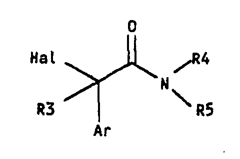 mit einem Amin 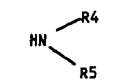 umsetzt; oder
c) eine Verbindung I, in der R⁵ H ist N-alkyliert;
und eine so erhaltene Verbindung als freie Verbindung oder als deren pharmazeutisch annehmbares Salz isoliert.

23. Pharmazeutische Zubereitung enthaltend eine Verbindung nach einem der Ansprüche 1 bis 21 und pharmazeutisch annehmbare Träger und Excipienten.

24. Verwendung einer Verbindung nach einem der Ansprüche 1 bis 22 für die Herstellung einer pharmazeutischen Zubereitung zur Therapie von und zur Vorbeugung gegenüber Neurokinin-vermittelten Krankheiten.

25. Eine Verbindung nach einem der Ansprüche 1 bis 22 zur Therapie von und zur Vorbeugung gegenüber Neurokinin-vermittelten Krankheiten.

## Claims

1. Arylglycinamide derivatives of general formula I 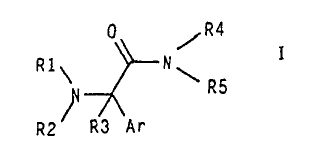 or the pharmaceutically acceptable salts thereof, wherein
Ar denotes unsubstituted or mono- to penta-substituted phenyl, or unsubstituted or mono- or disubstituted naphthyl [wherein the substituents of the phenyl and naphthyl independently of one another denote halogen (F, Cl, Br, I), (C₁₋₄)alkyl, O-(C₁₋₄)alkyl, CF₃, OCF₃ or NR¹²R¹³ (wherein R¹² and R¹³ independently of one another denote H, methyl or acetyl)] or Ar is phenyl substituted by -O-CH₂-O- or -O-(CH₂)₂-O-;
R¹ and R² together with the N to which they are bound denote a ring of the formula 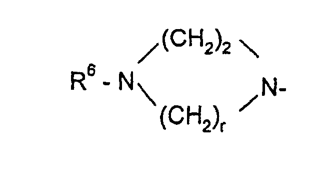 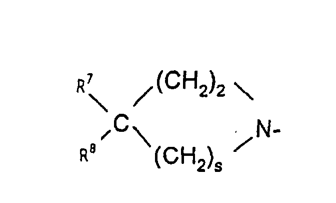 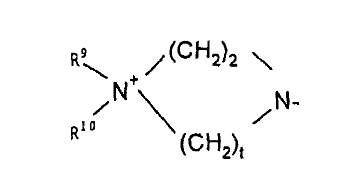 or 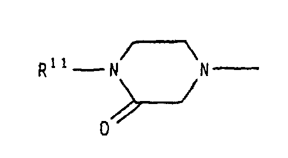 wherein
r, s and t are 2 or 3;
R⁶ denotes
H,
(C₁₋₅) alkyl,
(C₃₋₅) alkenyl,
propynyl,
hydroxy (C₂₋₄) alkyl,
methoxy (C₂₋₄) alkyl,
di (C₁₋₃) alkylamino(C₂₋₄) alkyl,
amino(C₂₋₄)alkyl,
amino,
di (C₁₋₃) alkylamino,
monofluoro- to perfluoro (C₁₋₂) alkyl,
N-methylpiperidinyl,
pyridyl,
pyrimidinyl,
pyrazinyl,
pyridazinyl
or the group -CH₂-C(O)NR¹⁴R¹⁵,
wherein
R¹⁴ is H or (C₁₋₄)alkyl and
R¹⁵ is H, (C₁₋₄)alkyl, (C₃₋₆)cycloalkyl, hydroxy(C₂₋₄)alkyl, alkoxy(C₂₋₃)alkyl, phenyl(C₁₋₄)alkyl, or R¹⁴ and R¹⁵ together with the N to which they are bound form a ring (1-pyrrolidinyl, piperidino, morpholino or 1-methylpiperazin-4-yl);
R⁷ has one of the definitions (a) to (d),
(a) hydroxy
(b) 4-piperidinopiperidyl,
(c) 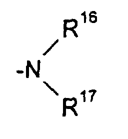 wherein R¹⁶ and R¹⁷ independently of one another denote
H,
(C₁₋₄)alkyl,
(C₃₋₆)cycloalkyl,
hydroxy (C₂₋₄) alkyl,
(C₁₋₃) alkoxy (C₂₋₄) alkyl,
phenyl (C₁₋₄) alkyl or
di (C₁₋₃) alkylamino (C₂₋₄) alkyl,
or if R¹⁶ is H or (C₁₋₄)alkyl,
R¹⁷ may also be -CH₂C(O)NR¹⁸R¹⁹, wherein R¹⁸ and R¹⁹ are defined as R¹⁴ and R¹⁵ hereinbefore;
(d) 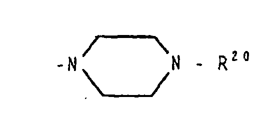 wherein R²⁰ denotes
H,
(C₁₋₄)alkyl,
(C₄₋₆)cycloalkyl or
-CH₂C(O)NR²¹R²²,
wherein R²¹ and R²² are defined as R¹⁴ and R¹⁵
hereinbefore;
R⁸ is H
R⁹ and R¹⁰ independently of each other denote (C₁₋₄)alkyl;
R¹¹ denotes
H,
(C₁₋₅) alkyl,
(C₃₋₅) alkenyl,
propynyl,
hydroxy(C₂₋₄) alkyl,
methoxy (C₂₋₃) alkyl,
di (C₁₋₃)alkylamino (C₂₋₃) alkyl,
amino (C₂₋₃) alkyl,
amino,
di (C₁₋₃) alkylamino,
monofluoro- to perfluoro (C₁₋₂) alkyl,
N-methylpiperidinyl,
pyridyl,
pyrimidinyl,
pyrazinyl,
pyridazinyl
or the group -CH₂-C(O)NR²³R²⁴,
wherein R²³ and R²⁴ are defined as R¹⁴ and R¹⁵ hereinbefore;
R³ denotes H, (C₁₋₄)alkyl, unsubstituted or mono- to trisubstituted phenyl, wherein the substituents independently of one another denote halogen (F, Cl, Br, I), (C₁₋₄)alkyl, O-(C₁₋₄)alkyl, CF₃, OCF₃ or NR²⁵R²⁶ (wherein R²⁵ and R²⁶ independently of one another denote H, methyl or acetyl);
R⁴ denotes phenyl (C₁₋₄)alkyl or naphthyl (C₁₋₄)alkyl, wherein phenyl may be substituted by 1 to 3 substituents, wherein the substituents independently of one another denote halogen (F, Cl, Br, I), (C₁₋₄)alkyl, O-(C₁₋₄)alkyl, CF₃, OCF₃ or NR²⁷R²⁸ (wherein R²⁷ and R²⁸ independently of one another denote H, methyl or acetyl);
and
R⁵ denotes H, (C₁₋₄)alkyl, (C₃₋₆)cycloalkyl, CH₂COOH, CH₂C(O)NH₂, OH or phenyl(C₁₋₄)alkyl.

2. Compound according to claim 1 wherein
Ar denotes unsubstituted or mono- or disubstituted phenyl, or unsubstituted naphthyl, or Ar is phenyl substituted by -O-CH₂-O- or -O-(CH₂)₂-O-;
R¹ and R² together with the N to which they are bound denote a ring of the formula 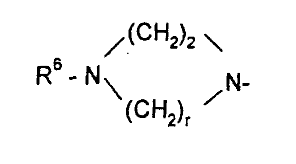 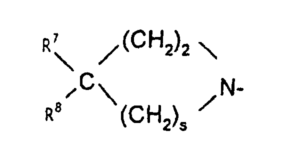 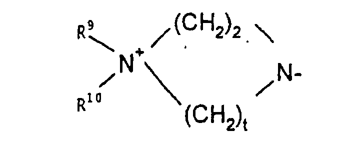 or 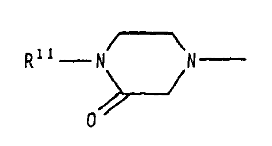 wherein
r is 2 or 3 and
s and t are 2;
R⁶, R⁷, R⁸, R⁹, R¹⁰ and R¹¹ are defined as in claim 1;
R³ is H or (C₁₋₄)alkyl,
R⁴ denotes phenyl (C₁₋₄) alkyl or naphthyl (C₁₋₄) alkyl,
wherein phenyl may be substituted by 1 or 2 substituents, wherein the substituents independently of one another are halogen (F, Cl, Br, I), (C₁₋₄)alkyl, O-(C₁₋₄)alkyl, CF₃ or OCF₃;
and
R⁵ denotes H, (C₁₋₄)alkyl, (C₃₋₆)cycloalkyl, OH or phenyl (C₁₋₄) alkyl.

3. Compound according to claim 1 or 2, wherein
Ar is unsubstituted or mono- or disubstituted phenyl, or unsubstituted naphthyl [wherein the substituents of the phenyl independently of one another denote halogen (F, Cl, Br, I), methyl, methoxy, CF₃ or OCF₃] or Ar is phenyl substituted by -O-CH₂-O- or -O-(CH₂)₂-O-.

4. Compound according to claim 3, wherein Ar is phenyl, naphthyl, phenyl substituted by methoxy or halogen in position 3 and/or 4 or phenyl wherein positions 2 and 3 or 3 and 4 are linked by -O-CH₂-O-.

5. Compound according to claim 4, wherein Ar is phenyl, phenyl substituted by methoxy in positions 3 and 4 or phenyl in which positions 3 and 4 or 2 and 3 are linked by -O-CH₂-O-.

6. Compound according to one of claims 1 to 5, wherein in the ring 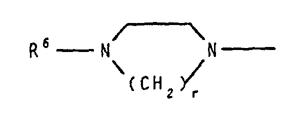 r is 2 or 3 and
R⁶ denotes
H,
(C₁₋₅)alkyl,
(C₃₋₅) alkenyl,
propynyl,
hydroxy (C₂₋₄) alkyl,
methoxy (C₂₋₄) alkyl,
di (C₁₋₃) alkylamino (C₂₋₄)alkyl,
amino (C₂₋₄) alkyl,
amino,
di (C₁₋₃) alkylamino,
monofluoro- to perfluoro(C₁₋₂)alkyl,
N-methylpiperidinyl,
pyridyl,
pyrimidinyl,
or

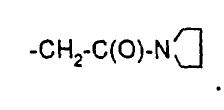

7. Compound according to claim 6, wherein
r is 3 and R⁶ is methyl.

8. Compound according to claim 6, wherein
r is 2 and
R⁶ is
H,
(C₁₋₄)alkyl,
propenyl,
propynyl,
hydroxy (C₂₋₃) alkyl,
methoxyethyl,
di (C₁₋₂)alkylamino(C₂₋₃) alkyl,
aminoethyl,
amino,
dimethylamino,
CH₂CF₃,
N-methylpiperidinyl,
pyridyl,
pyrimidinyl
or

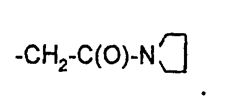

9. Compound according to claim 8, wherein
r is 2 and
R⁶ is H, (C₁₋₃)alkyl, allyl, 2-propynyl, -CH₂CH₂OCH₃, -CH₂CH₂N(CH₃)₂, N-methylpiperidinyl, 2-pyrimidinyl
or

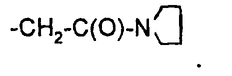

10. Compound according to claim 9, wherein
r is 2 and
R⁶ is H, CH₃, C₃H₇, CH(CH₃)₂, CH₂CH₂OCH₃ or CH₂CH₂N(CH₃)₂.

11. Compound according to one of claims 1 to 5, wherein R¹ and R² together with the N to which they are bound form the ring 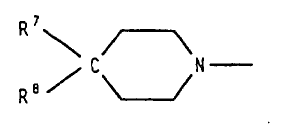 wherein R⁸ is H and
R⁷ is OH 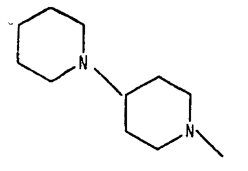 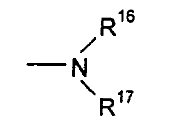 wherein R¹⁶ and R¹⁷ independently of one another denote:
H
(C₁₋₃)alkyl, 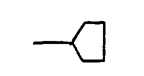 (CH₂)ₙOH wherein n is 2, 3 or 4
(CH₂)₂OCH₃
-(CH₂)ₙPh wherein n is 2 or 4
(CH₂)₂N(CH₃)₂ 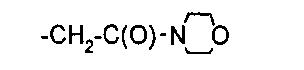 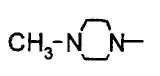 or

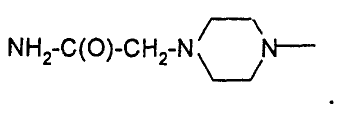

12. Compound according to claim 11, wherein
R¹⁶ and R¹⁷ are both CH₃ or C₂H₅ or
R¹⁶ is H or CH₃ and R¹⁷ is
(C₁₋₃)alkyl, 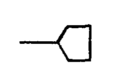 (CH₂)₂OH,
(CH₂)₄OH or

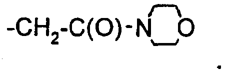

13. Compound according to claim 11 wherein
R⁷ denotes 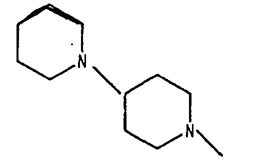 N(CH₃)₂ 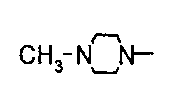 or

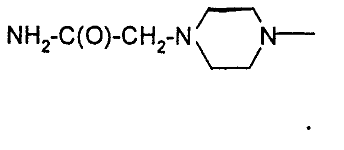

14. Compound according to claim 11,
wherein
R¹ and R² together with the N to which they are bound form the ring 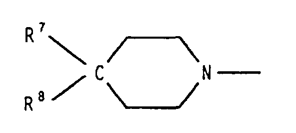 wherein
(a) R⁸ is H and
R⁷ is 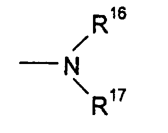 wherein R¹⁶ and R¹⁷ both represent CH₃, C₂H₅ or CH₂CH₂OH or R¹⁶ is H or CH₃ and R¹⁷ is (C₁₋₃)alkyl, 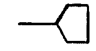 (CH₂)₂OH or
(CH₂)₄OH
or
(b) R⁸ is H and
R⁷ denotes

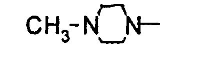

15. Compound according to one of claims 1 to 5, wherein R¹ and R² together with the N to which they are bound form the ring

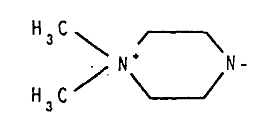

16. Compound according to one of claims 1 to 5, wherein R¹ and R² together with the N to which they are bound form the ring 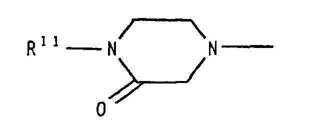 wherein R¹¹ is H or (C₁₋₃)alkyl.

17. Compound according to claim 16, wherein
R¹¹ is -CH(CH₃)₂.

18. Compound according to one of claims 1 to 17,
wherein R³ is H.

19. Compound according to one of claims 1 to 18,
wherein
R⁴ denotes phenyl(C₁₋₄)alkyl, wherein phenyl may be substituted by 1 or 2 substituents, in which the substituents independently of one another are halogen (F, Cl, Br, I), (C₁₋₄)alkyl, O-(C₁₋₄)alkyl, CF₃ or OCF₃;
and
R⁵ denotes H, (C₁₋₄)alkyl, (C₃₋₆)cycloalkyl, -OH or phenyl (C₁₋₄) alkyl.

20. Compound according to claim 19, wherein
R⁴ denotes phenyl(C₂₋₄)alkyl, wherein the substituents are in positions 3 and/or 5 of the phenyl ring and
R⁵ is H, methyl, OH or phenethyl.

21. Compound according to claim 20, wherein R⁴ is 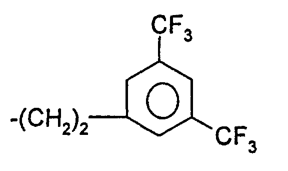 and R⁵ is methyl.

22. Process for preparing a compound of general formula I according to one of claims 1 to 21, **characterised in that**
a) an acid 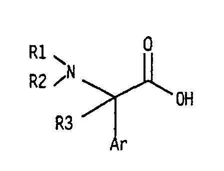 or a halide or alkylester thereof is reacted with an amine 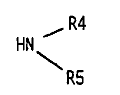
b) an α-haloarylacetamide 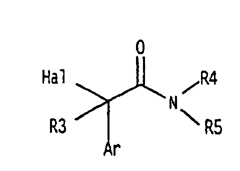 is reacted with an amine 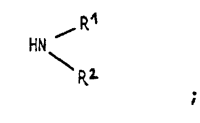 or
c) a compound I wherein R⁵ is H is N-alkylated;
and a compound thus obtained is isolated as a free compound or as a pharmaceutically acceptable salt thereof.

23. Pharmaceutical preparation containing a compound according to one of claims 1 to 21 and pharmaceutically acceptable carriers and excipients.

24. Use of a compound according to one of claims 1 to 21 for preparing a pharmaceutical preparation for the treatment and prevention of neurokinin-mediated diseases.

25. A compound according to one of claims 1 to 21 for the treatment and prevention of neurokinin-mediated diseases.

## Revendications

1. Dérivés d'arylglycinamide de formule générale I 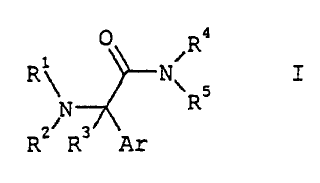 ou leurs sels pharmaceutiquement acceptables,
où
Ar est phényle non substitué ou substitué 1 à 5 fois, ou naphtyle non substitué ou substitué 1 ou 2 fois [où les substituants du phényle et du naphtyle sont indépendamment les uns des autres halogène (F, Cl, Br, I) , alkyle en C₁-C₄, O-alkyle en C₁-C₄, CF₃, OCF₃ ou NR¹²R¹³ (où R¹² et R¹³ sont indépendamment l'un de l'autre H, méthyle ou acétyle)] ou Ar est phényle substitué par -O-CH₂-O- ou -O-(CH₂)₂-O- ;
R¹ et R² forment avec le N auquel ils sont liés un cycle de formule 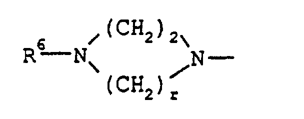 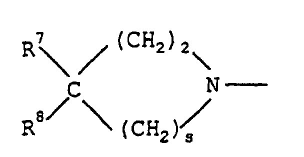 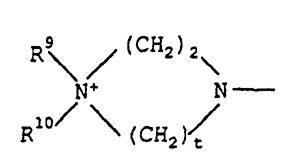 ou 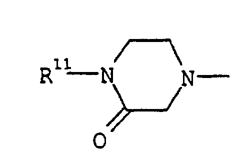 où
r, s et t sont 2 ou 3 ;
R⁶ représente
H,
alkyle en C₁-C₅,
alcényle en C₃-C₅,
propynyle,
hydroxyalkyle en C₂-C₄,
méthoxy-alkyle en C₂-C₄,
dialkyle en C₁-C₃-aminoalkyle en C₂-C₄,
aminoalkyle en C₂-C₄,
amino,
dialkyle en C₁-C₃-amino,
monofluoro- à perfluoroalkyle en C₁-C₂,
N-méthylpipéridinyle,
Pyridyle,
Pyrimidinyle,
Pyrazinyle,
Pyridazinyle
ou le groupe -CH₂-C(O)NR¹⁴R¹⁵,
où
R¹⁴ est H ou alkyle en C₁-C₄ et
R¹⁵ est alkyle en C₁-C₄, cycloalkyle en C₃-C₆, hydroxyalkyle en C₂-C₄, alcoxy-alkyle en C₂-C₃, phényl-alkyle en C₁-C₄, ou bien R¹⁴ et R¹⁵ forment avec le N auquel ils sont liés un cycle (1-pyrrolidinyle, pipéridino, morpholino ou 1-méthylpipérazin-4-yle) ;
R⁷ a l'une des significations (a) à (d),
(a) hydroxyle
(b) 4-pipéridinopipéridyle,
(c) 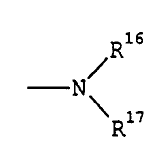 où R¹⁶ et R¹⁷ sont indépendamment l'un de l'autre
H,
alkyle en C₁-C₄,
cycloalkyle en C₃-C₆,
hydroxyalkyle en C₂-C₄,
alcoxy en C₁-C₃-alkyle en C₂-C₄,
phényl-alkyle en C₁-C₄ ou
dialkyle en C₁-C₃-aminoalkyle en C₂-C₄,
ou bien, quand R¹⁶ est H ou alkyle en C₁-C₄,
R¹⁷ peut aussi être -CH₂C(O)NR¹⁸R¹⁹, où R¹⁸ et R¹⁹ sont définis comme R¹⁴ et R¹⁵ ci-dessus ;
(d) 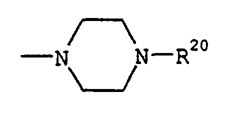 où R²⁰ est
H
alkyle en C₁-C₄,
cycloalkyle en C₄-C₆ ou
-CH₂C(O)NR²¹R²²,
où R²¹ et R²² sont définis comme R¹⁴ et R¹⁵ ci-dessus ;
R⁸ est H
R⁹ et R¹⁰ sont indépendamment l'un de l'autre
alkyle en C₁-C₄ ;
R¹¹ représente
H,
alkyle en C₁-C₅,
alcényle en C₃-C₅,
propynyle,
hydroxyalkyle en C₂-C₄,
méthoxy-alkyle en C₂-C₃,
dialkyle en C₁-C₃-aminoalkyle en C₂-C₃,
aminoalkyle en C₂-C₃,
amino,
dialkyle en C₁-C₃-amino,
monofluoro- à perfluoroalkyle en C₁-C₂,
N-méthylpipéridinyle,
pyridyle,
pyrimidinyle,
pyrazinyle,
pyridazinyle
ou le groupe -CH₂-C(O)NR²³R²⁴,
où R²³ et R²⁴ sont définis comme R¹⁴ et R¹⁵ ci-dessus ;
R³ est H, alkyle en C₁-C₄, phényle non substitué ou substitué 1-3 fois, où les substituants sont indépendamment les uns des autres halogène (F, Cl, Br, I), alkyle en C₁-C₄, O-alkyle en C₁-C₄, CF₃, OCF₃ ou NR²⁵R²⁶ (où R²⁵ et R²⁶ sont indépendamment l'un de l'autre H, méthyle ou acétyle) ;
R⁴ représente phényl-alkyle en C₁-C₄ ou naphtyl-alkyle en C₁-C₄, où phényle peut être substitué par 1 à 3 substituants, où les substituants sont indépendamment les uns des autres halogène (F, Cl, Br, I), alkyle en C₁-C₄, O-alkyle en C₁-C₄, CF₃, OCF₃ ou NR²⁷R²⁸ (où R²⁷ et R²⁸ sont indépendamment l'un de l'autre H, méthyle ou acétyle) ;
et
R⁵ représente H, alkyle en C₁-C₄, cycloalkyle en C₃-C₆, CH₂COOH, CH₂C(O)NH₂, OH ou phényl-alkyle en C₁-C₄.

2. Composé selon la revendication 1
où
Ar est phényle non substitué ou substitué 1 à 2 fois, ou naphtyle non substitué, ou Ar est phényle substitué par -O-CH₂-O- ou -O-(CH₂)₂-O- ;
R¹ et R² forment avec le N auquel ils sont liés un cycle de formule 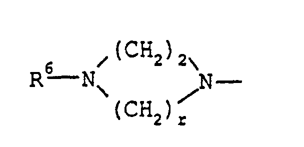 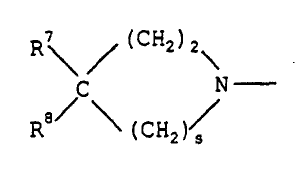 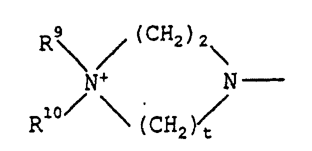 ou 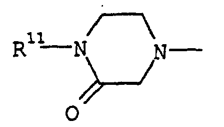 où
r est 2 ou 3 et
s et t sont 2 ;
R⁶, R⁷, R⁸, R⁹, R¹⁰ et R¹¹ sont définis comme dans la revendication 1 ;
R³ est H ou alkyle en C₁-C₄,
R⁴ représente phényl-alkyle en C₁-C₄ ou naphtyl-alkyle en C₁-C₄, où phényle peut être substitué par 1 ou 2 substituants, où les substituants sont indépendamment l'un de l'autre halogène (F, Cl, Br, I), alkyle en C₁-C₄, O-alkyle en C₁-C₄, CF₃ ou OCF₃ ;
et
R⁵ représente H, alkyle en C₁-C₄, cycloalkyle en C₃-C₆, OH ou phényl-alkyle en C₁-C₄.

3. Composé selon la revendication 1 ou 2 où
Ar est phényle non substitué ou substitué 1 ou 2 fois,
ou naphtyle non substitué [où les substituants du phényle sont indépendamment l'un de l'autre halogène (F, Cl, Br, I), méthyle, méthoxy, CF₃ ou OCF₃] ou Ar est phényle substitué par -O-CH₂-O- ou -O-(CH₂)₂-O-.

4. Composé selon la revendication 3 où Ar est phényle, naphtyle, phényle substitué en position 3 et/ou 4 par méthoxy ou halogène ou phényle dont les positions 2 et 3, ou 3 et 4 sont liées par -O-CH₂-O-.

5. Composé selon la revendication 4 où Ar est phényle, phényle substitué dans les positions 3 et 4 par méthoxy ou phényle dont les positions 3 et 4 ou 2 et 3 sont liées par -O-CH₂-O-.

6. Composé selon l'une des revendications 1 à 5 où, dans le cycle 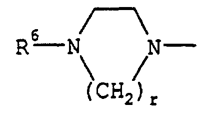 r est 2 ou 3, et
R⁶ est
H,
alkyle en C₁-C₅,
alcényle en C₃-C₅,
propynyle,
hydroxyalkyle en C₂-C₄,
méthoxy-alkyle en C₂-C₄,
dialkyle en C₁-C₃-aminoalkyle en C₂-C₄,
aminoalkyle en C₂-C₄,
amino,
dialkyle en C₁-C₃-amino,
monofluoro- à perfluoroalkyle en C₁-C₂,
N-méthylpipéridinyle,
pyridyle,
pyrimidinyle,
ou

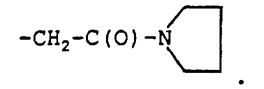

7. Composé selon la revendication 6 où r est 3 et R⁶ est méthyle.,

8. Composé selon la revendication 6 où
r est 2 et
R⁶ est
H,
alkyle en C₁-C₄,
propényle,
propynyle,
hydroxyalkyle en C₂-C₃,
méthoxyéthyle,
dialkyle en C₁-C₂-aminoalkyle en C₂-C₃,
aminoéthyle,
amino,
diméthylamino,
CH₂CF₃,
N-méthylpipéridinyle,
pyridyle,
pyrimidinyle,
ou

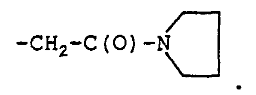

9. Composé selon la revendication 8 où
r est 2 et
R⁶ est H, alkyle en C₁-C₃, allyle, 2-propynyle, -CH₂CH₂OCH₃, -CH₂CH₂N(CH₃)₂, N-méthylpipéridinyle, 2-pyrimidinyle ou

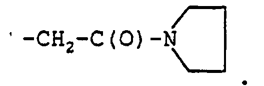

10. Composé selon la revendication 9 où
r est 2 et
R⁶ est H, CH₃, C₃H₇, CH(CH₃)₂, CH₂CH₂OCH₃ ou CH₂CH₂N(CH₃)₂.

11. Composé selon l'une quelconque des revendications 1 à 5 où R¹ et R² forment avec le N auquel ils sont liés le cycle 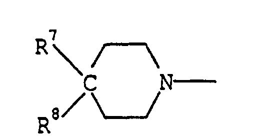 où R⁸ est H et
R⁷ est
OH 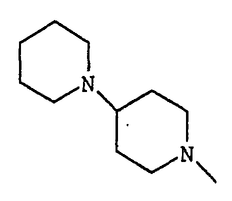 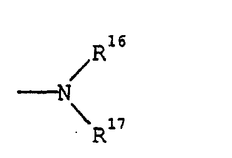 où R¹⁶ et R¹⁷ sont indépendamment l'un de l'autre :
H
alkyle en C₁-C₃ 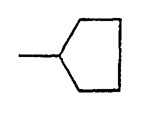 (CH₂)ₙOH où n est 2, 3 ou 4
(CH₂)₂OCH₃
-(CH₂)ₙPh où n est 2 ou 4
(CH₂)₂N(CH₃)₂ 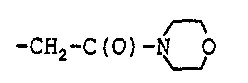 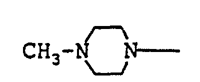 ou

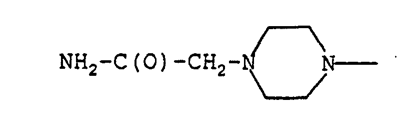

12. Composé selon la revendication 11 où R¹⁶ et R¹⁷ sont tous les deux CH₃ ou C₂H₅ ou
R¹⁶ est H ou CH₃ et R¹⁷ est
alkyle en C₁-C₃ 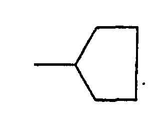 (CH₂)₂OH
(CH₂)₄OH ou

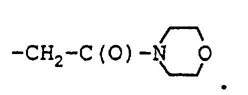

13. Composé selon la revendication 11 où R⁷ est 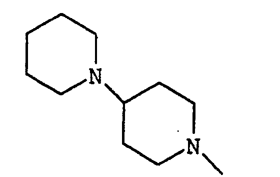 N(CH₃)₂ 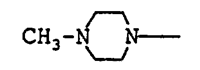 ou

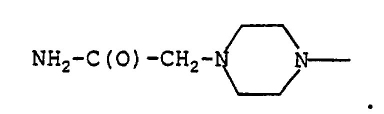

14. Composé selon la revendication 11 où R¹ et R² forment avec le N auquel ils sont liés le cycle 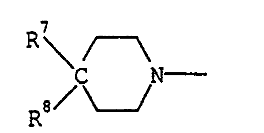 où
(a) R⁸ est H et
R⁷ est 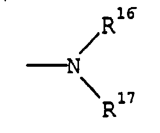 où
R¹⁶ et R¹⁷ sont tous les deux CH₃, C₂H₅ ou
CH₂CH₂OH ou
R¹⁶ est H ou CH₃ et R¹⁷ est
alkyle en C₁-C₃ 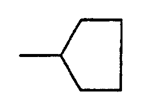 (CH₂)₂OH ou
(CH₂)₄OH
ou
(b) R⁸ est H et
R⁷ est

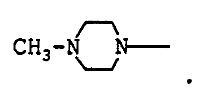

15. Composé selon l'une des revendications 1 à 5 où R¹ et R² forment avec le N auquel ils sont liés le cycle

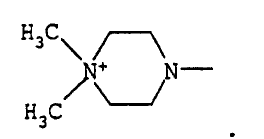

16. Composé selon l'une des revendications 1 à 5 où R¹ et R² forment avec le N auquel ils sont liés le cycle 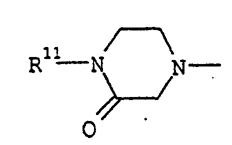 où R¹¹ est H ou alkyle en C₁-C₃.

17. Composé selon la revendication 16 où R¹¹ est -CH(CH₃)₂.

18. Composé selon l'une des revendications 1 à 17 où R³ est H.

19. Composé selon l'une des revendications 1 à 18 où R⁴ représente phényl-alkyle en C₁-C₄ où phényle peut être substitué par 1 ou 2 substituants, où les substituants sont indépendamment l'un de l'autre halogène (F, Cl, Br, I), alkyle en C₁-C₄, O-alkyle en C₁-C₄, CF₃ ou OCF₃ ;
et
R⁵ représente H, alkyle en C₁-C₄, cycloalkyle en C₃-C₆, -OH ou phényl-alkyle en C₁-C₄.

20. Composé selon la revendication 19 où
R⁴ représente phényl-alkyle en C₂-C₄ où les substituants sont dans les positions 3 et/ou 5 du cycle phényle et R⁵ est H, méthyle, OH ou phénéthyle.

21. Composé selon la revendication 20 où R⁴ est 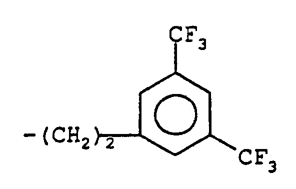 et R⁵ est méthyle.

22. Procédé de préparation d'un composé de formule générale I selon l'une des revendications 1 à 21 **caractérisé en ce que**
a) on fait réagir un acide 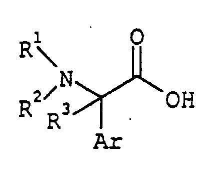 ou son halogénure ou alkylester avec une amine 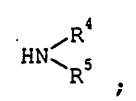
b) on fait réagir un α-halogénoarylacétamide 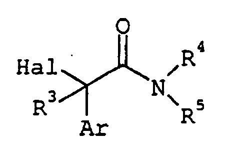 avec une amine 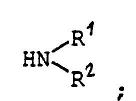 ou
c) on N-alkyle un composé I où R⁵ est H ;
et on isole un composé ainsi obtenu sous forme de composé libre ou sous forme de son sel pharmaceutiquement acceptable.

23. Préparation pharmaceutique contenant un composé selon l'une des revendications 1 à 21 et des supports et excipients pharmaceutiquement acceptables.

24. Utilisation d'un composé selon l'une des revendications 1 à 21 pour la production d'une préparation pharmaceutique pour la thérapie et la prévention des maladies dues à des neurokinines.

25. Composé selon l'une des revendications 1 à 2.1 pour la thérapie et là prévention des maladies dues à des neurokinines.
